# EUROPÄISCHE PATENTSCHRIFT

(11) **EP 1 725 210 B1**
(45) Veröffentlichungstag und Bekanntmachung des Hinweises auf die Patenterteilung: **22.09.2010**
(21) Anmeldenummer: 05715971.7
(22) Anmeldetag: 11.03.2005
(51) Int. Cl.: A61K 8/97, A61Q 15/00

(54) **VERWENDUNG VON EINEM WASSER/ETHANOL-EXTRAKT AUS HIBISCUS SABDARIFFA ZUR BEHANDLUNG VON KÖRPERGERUCH**
USE OF A WATER/ETHANOL EXTRACT OF SUDANESE TEA (HIBISCUS) FOR THE TREATMENT OF BODY ODOURS
UTILISATION D'UN EXTRAIT EAU/ETHANOL D'HIBISCUS POUR TRAITER LES ODEURS CORPORELLES

(30) Priorität: 18.03.2004 DE 102004013694; 07.07.2004 DE 102004032734
(43) Veröffentlichungstag der Anmeldung: 29.11.2006
(73) Patentinhaber: Henkel AG & Co. KGaA, 40589 Düsseldorf (DE)
(72) Erfinder: BOCKMÜHL, Dirk, 42113 Wuppertal (DE); HÖHNE, Heide-Marie, 50189 Elsdorf (DE); JASSOY, Claudia, 40237 Düsseldorf (DE); SCHOLTYSSEK, Regine, 40882 Mettmann (DE); BANOWSKI, Bernhard, 40597 Düsseldorf (DE); WADLE, Armin, 40699 Erkrath (DE); SÄTTLER, Andrea, 40225 Düsseldorf (DE); NIEVELER, Silke, 41236 Mönchengladbach (DE); BREVES, Roland, 40822 Mettmann (DE)
(86) Internationale Anmeldenummer: PCT/EP2005/002606
(87) Internationale Veröffentlichungsnummer: WO 2005/092279

(56) Entgegenhaltungen:
- EP-A- 0 126 944
- EP-A- 0 297 310
- EP-A- 0 599 433
- WO-A-91/18586
- WO-A-98/22081
- WO-A-2005/011716
- WO-A-2005/033331
- DE-A1- 2 728 921
- DE-A1- 19 503 423
- FR-A- 2 713 086
- "PRODUCT LIST 2004" [Online] Januar 2004 (2004-01), COSMETOCHEM INTERNATIONAL LTD , XP002330972 Gefunden im Internet: URL:www.cosmetochem.ch> [gefunden am 2005-06-08] Seite 8 Seite 9 Seite 12 Seite 13 Seite 16 Seite 17
- ANDREW F. ALEXIS, VALERIE A. JONES, MA, METTHEW J. STILLER, MD: "Potential therapeutic application of tea in dermatology" INTERNATIONAL JOURNAL OF DERMATOLOGY, Bd. 38, 1999, XP002330969
- GROUPE SOLABIA: LE PETIT BOTANISTE, [Online] Nr. 4, Juni 1998 (1998-06), XP002330970
- AFTAB ALAM, NAGHMA KHAN, SONIA SHARMA, MOHAMMAD SALEEM, SARWAT SULTANA: "Chemopreventive effect of Vtis Vinifera Extract on 12-o-tetradecanoyl-13-phorbol acetate-induced cutaneous oxidative stress ans tumor promotion in murine skin" PHARMACOLOGICAL RESEARCH, Bd. 46, 6. Dezember 2002 (2002-12-06), XP002330971
- GORA J ET AL: "CHEMICAL SUBSTANCES FROM INFLORESCENCES OF ARNICA MONTANA L. AND CALENDULA OFFICINALIS L. SOLUBLE IN ISOPROPYL MYRISTATE AND PROPYLENE GLYCOL" HERBA HUNGARICA, BUDAPEST, HU, Bd. 19, Nr. 1, 1980, Seiten 165-171, XP000604334 ISSN: 0018-0580

## Beschreibung

Gegenstand der vorliegenden Erfindung ist die Verwendung eines Pflanzenextraktes, des auf der Haut, insbesondere im axillaren Bereich, präbiotisch wirksam ist zur Bekämpfung von Körpergeruch.

Körpergeruch entsteht durch den Abbau von Schweißbestandteilen durch Bakterien der Hautflora. Aus diesem Grund werden seit langem antibakterielle Substanzen in Deodorantien verwendet. Die Verwendung von unselektiv antibakteriell wirksamen Substanzen hat allerdings den Nachteil, dass auch Bakterien, die keinen Geruch verursachen, gehemmt oder abgetötet werden. Die Schutzfunktion für die Haut, die von diesen Bakterien ausgeht, wird durch die Anwendung unselektiv antibakterieller Wirkstoffe somit zerstört. Zusätzlich ist bekannt, dass viele antibakterielle Wirkstoffe schlecht gegen Körpergeruch wirken. Daraus lässt sich schließen, dass durch die eingesetzten antibakteriellen Wirkstoffe die geruchsbildenden Bakterien nicht oder nur ungenügend gehemmt werden bzw. dass möglicherweise Zusammenhänge in der bakteriellen Lebensgemeinschaft existieren (z.B. bislang unbekannte geruchsproduzierende Arten), deren Auswirkungen auf die Bildung von Körpergeruch von den eingesetzten Substanzen nicht erfasst werden.

Es besteht daher Bedarf, Mittel zur Bekämpfung von Körpergeruch, insbesondere für den axillaren Bereich, zur Verfügung zu haben, insbesondere solche, die besser gegenüber Körpergeruch wirksam sind, als die bislang bekannten Mittel. Diese Mittel sollten ferner vorteilhafterweise selektiv gegen die geruchsbildenden Mikroorganismen wirksam sein.

Aufgabe der vorliegenden Erfindung war es hierbei vorzugsweise, Mittel zur Verfügung zu stellen, die auf der Haut das Wachstum und/oder die physiologische Aktivität von wünschenswerten Keimen der Hautflora gegenüber dem Wachstum und/oder der physiologischen Aktivität von unerwünschten Keimen der Hautflora fördern. Solche Substanzen werden auch als "präbiotisch" bezeichnet.

Aufgabe der vorliegenden Erfindung war es hierbei insbesondere, Substanzen ausfindig zu machen, die das Wachstum und/oder die physiologische Aktivität geruchsneutraler Keime fördern und/oder das Wachstum und/oder die physiologische Aktivität geruchsbildender bzw. unangenehmen Geruch bildender Keime hemmen und auf diese Weise insbesondere im Achselbereich einen präbiotischen Effekt bewirken können.

Für den Achselbereich sind bislang noch keine präbiotisch wirksamen Substanzen beschrieben worden, insbesondere keine Substanzen, die selektiv das Wachstum und/oder die physiologische Aktivität von geruchsneutralen Keimen auf Kosten des Wachstums und/oder der physiologischen Aktivität von geruchsbildenden bzw. von unangenehmen Geruch verursachenden Keimen fördern.

Der Nachweis eines präbiotischen Effektes von Substanzen ist bislang im wesentlichen beschränkt auf das Intestum. So ist in verschiedenen Publikationen die Verwendung von Substanzen, die förderlich auf das Wachstum wünschenswerter Darmbakterien sind, beschrieben. Ahn et al. (1990) Microbial Ecology in Health and Disease 3, 223-229, beschreibt hierbei insbesondere auch die Verwendung eines Ginseng-Extraktes.

Für die Haut wurde bislang lediglich berichtet, dass ein Oligosaccharid einen präbiotischen Effekt bewirkt (Werbebroschüre über BioEcolia® der Solabia Group, Frankreich), indem es selektiv von saprophyten Bakterien bevorzugt verwertet werden kann. Es wurde hierbei gezeigt, dass das verwendete Oligosaccharid das Wachstum von *Micrococcus kristinae* sowohl gegenüber dem Wachstum von *Staphylococcus aureus* als auch gegenüber dem Wachstum von *Corynebakterium xerosis* fördert.

Desweiteren wurde in EP1050300 berichtet, dass eine Mischung aus Farnesol und Xylitol als präbiotische Substanz verwendet werden kann, da diese Mischung selektiv antibakteriell gegenüber *S. aureus* wirkt und sich so der konkurrierende Keim *S. epidermidis* besser vermehren kann.

Als antibakterielle und desodorierende Wirkstoffe sind Extrakte aus Citrusfruchtsamen im Stand der Technik aus den Dokumenten EP 911019 und JP 9040516 bekannt, allerdings ist für diese keine präbiotische Wirkung beschrieben worden.

FR 2712086 A1 betrifft die Verwendung eines Polyols zur Hemmung des Wachstums unerwünschter Hautkeime und zur Förderung erwünschter Hautkeime und dessen Verwendung zur Wiederherstellung des Gleichgewichts der Hautflora. Kosmetische Zusammensetzungen mit Hibiscus sabdariffa-Extrakt waren bekannt aus der Produktliste 2004 der Firma Cosmetochem International Ltd. von Januar 2004. Die Zeitschrift "Le petit botaniste. Nr. 4, Juni 1998, offenbart die Verwendung von Hibiscus sabdariffa-Extrakten als Tee, im Nahrungsmittelbereich und zur Pflege öliger Haut, zur Antiage-Pflege, als Venentonikum und für Schlankheitsmittel.

Als Voraussetzung zur Lösung der erfindungsgemäßen Aufgabe wurden Untersuchungen angestellt, um zu ermitteln, welche Unterschiede im Mikrofloraprofil zwischen stark und schwach riechenden, insbesondere männlichen, Probanden auftreten, um hierdurch Keime ausfindig zu machen, die für die Geruchsbildung verantwortlich sind. Es wurde hierbei festgestellt, dass Probanden mit starkem bzw. unangenehmen Körpergeruch eine andere Zusammensetzung der bakteriellen Mikroflora aufweisen als Probanden mit schwachem Körpergeruch. Durch den Einsatz molekularbiologischer Methoden konnten erfindungsgemäß folgende Merkmale für Personen mit starkem Körpergeruch als typisch herausgefunden werden:
a) verminderter Anteil an *Staphylococcus epidermidis*
b) erhöhter Anteil an *Staphylococcus hominis*
c) leicht erhöhter Anteil an *Anaerococcus octavius*
d) leicht erhöhter Anteil an bestimmten *Corynebacterium Spezies*

Insbesondere das Verhältnis zwischen den beiden *Staphylococcus*-Spezies scheint dabei von besonderer Bedeutung zu sein. Eine mögliche Bedeutung des Staphylokokken-Verhältnisses auf den Körpergeruch ist im Stand der Technik bislang nicht beschrieben; ebenso wenig eine mögliche Bedeutung von Anaerokokken für den Körpergeruch. Im Stand der Technik ist hingegen bereits beschrieben, dass Corynebakterien und Mikrokokken in die Geruchsbildung involviert sein könnten.

Aufgrund der erfindungsgemäßen Ergebnisse wurde nun nach Substanzen gesucht, die das Mikrofloraprofil stark riechender Probanden bzw. von Probanden mit unangenehmen Körpergeruch verschieben hin zum Mikrofloraprofil schwach riechender Probanden, insbesondere indem sie selektiv das Wachstum geruchsneutraler Mikroorganismen, insbesondere geruchsneutraler Staphylokokken, vor allem von *S. epidermidis,* fördern und gleichzeitig das Wachstum von geruchsbildenden Staphylokokken, insbesondere von *S. hominis,* und/oder von gram-positiven anaeroben Kokken, insbesondere von Streptokokken, vor allem von *Anaerococcus octavius,* und/oder von geruchsbildenden Corynebakterien und/oder von geruchsbildenden Mikrokokken, vor allem von *Micrococcus luteus*, verhindern. Die Mindestanforderung an derartige Präbiotika war die Hemmung der geruchsbildenden Mikroorganismen ohne unmittelbaren Einfluss auf die geruchsneutralen bzw. die Förderung der geruchsneutralen Mikroorganismen ohne unmittelbaren Einfluss auf die geruchsbildenden.

Überraschenderweise wurden nun Substanzen gefunden, die auf der Haut einen präbiotischen Effekt bewirken, indem sie förderlich auf das Wachstum und/oder die physiologische Aktivität von *S. epidermidis* wirken und gleichzeitig hemmend auf das Wachstum und/oder die physiologische Aktivität von *S. hominis* wirken oder *S. hominis* zumindest nicht in seinem Wachstum fördern.

Hierbei handelt es sich um folgenden Pflanzenextrakt:
Extrakt aus Karkade (Hibiskus, Sudanesischer Tee, Hibiskus sabdariffa), (Fa. Cosmetochem; Wasser/Ethanol Trockenextrakt)

Unter präbiotischer Wirkung ist erfindungsgemäß zu verstehen, dass das Wachstum und/oder die physiologische Aktivität der erwünschten, insbesondere hautfreundlichen und/oder geruchsneutralen, Hautkeime bzw. Mikroflora gegenüber dem Wachstum und/oder der Überlebensfähigkeit der unerwünschten, insbesondere hautfeindlichen und/oder geruchsbildenden, Hautkeime bzw. Mikroflora gefördert wird. Dies kann sowohl dadurch erreicht werden, dass der Wirkstoff förderlich auf das Wachstum der erwünschten Hautkeime wirkt, ohne unmittelbar Einfluss auf das Wachstum der unerwünschten Hautkeime zu nehmen, als auch dadurch, dass der Wirkstoff hemmend auf das Wachstum der unerwünschten Hautkeime ist, ohne unmittelbar Einfluss auf das Wachstum der erwünschten Hautkeime zu nehmen. In einer erfindungsgemäß besonders bevorzugten und besonders überraschenden Ausführungsform jedoch wirkt der Wirkstoff förderlich auf das Wachstum der erwünschten Hautkeime und wirkt zugleich hemmend auf das Wachstum der unerwünschten Hautkeime.

Bei den unerwünschten Keimen kann es sich hierbei insbesondere handeln um hautfeindliche und/oder pathogene Keime und/oder um Keime, die im Vergleich zum Vorkommen beim gesunden Menschen, eine zu hohe Keimdichte aufweisen und dadurch gegebenenfalls einen unerwünschten und/oder pathogenen Effekt bewirken. Bei den unerwünschten Keimen kann es sich aber etwa auch handeln um geruchsbildende bzw. unangenehmen Geruch verursachende Keime.

Bei der erwünschten Mikroflora kann es sich hierbei entsprechend insbesondere handeln um hautfreundliche und/oder nicht-pathogene Keime, speziell der residenten Hautflora, saprophyte Keime oder aber, im Falle etwa von Körpergeruch, um Keime, die geruchsneutral sind, d.h. aus Schweißbestandteilen oder anderen Substanzen keine übelriechenden Verbindungen herstellen. Es ist hierbei insbesondere zu berücksichtigen, dass durch die Förderung des Wachstums der erwünschten Keime die unerwünschten Keime zurückgedrängt werden und umgekehrt durch die Hemmung des Wachstums der unerwünschten Keime die erwünschten Keime in ihrem Wachstum gefördert werden, so dass der präbiotische Effekt unterschiedlich bedingt sein kann.

Im Fall von (unangenehmen) Körpergeruch, und insbesondere Achselgeruch, handelt es sich bei den unerwünschten Keimen nicht notwendigerweise um pathogene Keime, sondern bei den geruchsbildenden Keimen kann es sich natürlich ebenso um an und für sich hautfreundliche Keime handeln. Im Falle von (unangenehmen) Körpergeruch sind die unerwünschten Keime also dadurch definiert, dass sie Körpergeruch verursachen. Eine präbiotische Substanz zeichnet sich in diesem Zusammenhang dadurch aus, dass sie das Wachstum der geruchsneutralen Keime auf Kosten des Wachstums der geruchsbildenden (unangnehmen Körpergeruch verursachenden) Keime fördert.

Unter "geruchsbildenden Keimen" bzw. "Geruchskeimen" sind erfindungsgemäß grundsätzlich solche Mikroorganismen zu verstehen, die bei Menschen mit Körpergeruch vermehrt auftreten. Bevorzugt handelt es sich hierbei um Mikroorganismen, die entweder selbst Substanzen produzieren bzw. die Bildung von Substanzen fördern, die einen unangenehmen Geruch verursachen. Des weiteren kann es sich hierbei auch um Mikroorganismen handeln, die nur mittelbar in die Bildung solcher Substanzen involviert sind, beispielsweise dadurch, dass sie eine Substanz produzieren bzw. die Bildung von Substanzen fördern, die durch andere Mikroorganismen zu unangenehm riechenden Substanzen umgesetzt werden können. Die geruchsbildenden Mikroorganismen müssen also erfindungsgemäß nicht notwendigerweise selbst den unangenehmen Geruch verursachen, sondern können auch in anderer Weise in den Metabolismus der Geruchsbildung involviert sein.

Gegenstand der vorliegenden Erfindung ist die Verwendung von auf der Haut präbiotisch wirksamen Substanzen in kosmetischen topischen Hautbehandlungsmitteln zur Förderung des Wachstums erwünschter Hautkeime und zur Hemmung des Wachstums unerwünschter Hautkeime zur Behandlung von Körpergeruch, insbesondere im Achselbereich, **dadurch gekennzeichnet, dass** die präbiotisch wirksame Substanz ein Wasser/Ethanol-Extrakt aus Hibiscus sabdariffa ist.

Die Verwendung kann hierbei vor allem jeweils in kosmetischen topischen Hautbehandlungsmitteln erfolgen, deren Zusammensetzung in dieser Beschreibung weiter spezifiziert ist.

Der präbiotisch wirksame Substanz ist hierbei vorzugsweise in einer Menge von 0,01 bis 20, besonders bevorzugt von 0,05 bis 10, vor allem von 0,1 bis 5, insbesondere von 0,1 bis 1,5 oder von 0,5 bis 2 Gew.-%, bezogen auf das Gesamtgewicht der Zusammensetzung, in der Zusammensetzung enthalten.

Bei dem Extrakt handelt es sich um einen Extrakt aus sudanesischem Tee (Karkade, Hibiskus, *Hibiscus sabdariffa*).

Die zuvor genannten Pflanzenextrakte sind beispielsweise erhältlich von den Firmen Cosmetochem (Deutschland) oder Rahn (Deutschland).

Die Herstellung des präbiotisch wirksamen Pflanzenextraktes kann grundsätzlich in jeder dem Fachmann bekannten Weise unter Verwendung jedes beliebigen Pflanzengewebes und unter Verwendung von Wasser/Ethanol als Extraktionsmittel erfolgen. So kann der Pflanzenextrakt beispielsweise durch Extraktion der gesamten Pflanze, durch Extraktion aus Blüten, Blättern, Samen, Wurzeln und/oder durch Extraktion aus dem Meristem der Pflanze erfolgen.

Nach der Extraktion können die Extrakte auch auf Träger aufgebracht werden, insbesondere um besser in Produkte eingearbeitet werden zu können. Erfindungsgemäß geeignete Träger sind beispielsweise Maltodextrin und Talk. In einer bevorzugten Ausführungsform der vorliegenden Erfindung richtet sich die Auswahl des Extraktes nach der Zubereitung, in der der Extrakt eingesetzt werden soll. So werden wässrige und alkoholische Extrakte, vorzugsweise eingesetzt in wässrigen oder alkoholischen Zubereitungen oder seifenhaltigen Stiften, Extrakte auf Maltodextrinträgern können sowohl in hydrophilen als auch in hydrophoben Produkten eingesetzt werden, Extrakte auf Talk-Trägern werden bevorzugt in hydrophoben Produkten eingesetzt.

Gegenstand der vorliegenden Erfindung sind demnach insbesondere auch die Verwendung gemäß Anspruch 1 von kosmetischen Zusammensetzungen, die erfindungsgemäß verwendete präbiotisch wirksame Pflanzenextrakte, insbesondere mindestens eines der zuvor genannten, auf Trägern, insbesondere auf Talk-Trägern oder auf Maltodextrin-Trägem, enthalten, deren Einsatz sich erfindungsgemäß als besonders vorteilhaft herausgestellt hat.

Bei dem Extrakt aus Hibiscus sabdariffa handelt es sich vorzugsweise um einen Wasser/Ethanol-Trockenextrakt oder um einen Wasser/Ethanol-Extrakt auf Maltodextrin- oder auf Talk-Trägern.

Die auf der Haut präbiotisch wirksamen Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch. Je nach Wahl der Extraktionsmittel kann es bevorzugt sein, den Pflanzenextrakt durch Zugabe eines Lösungsvermittlers zu stabilisieren. Als Lösungsvermittler geeignet sind z. B. Ethoxylierungsprodukte von gegebenenfalls gehärteten pflanzlichen und tierischen Ölen. Bevorzugte Lösungsvermittler sind ethoxylierte Mono-, Di- und Triglyceride von C₈₋₂₂-Fettsäuren mit 4 bis 50 Ethylenoxid-Einheiten, z. B. hydriertes ethoxyliertes Castoröl, Olivenölethoxylat, Mandelölethoxylat, Nerzölethoxylat, Polyoxyethylenglykolcapryl-/caprinsäureglycoride, Polyoxyethylenglycerinmono-laurat und Polyoxyethylenglykolkokosfettsäureglyceride.

Bei der erfindungsgemäß verwendeten kosmetischen Zusammensetzung kann es sich um jede beliebige Darreichungsform handeln, beispielsweise um eine feste oder flüssige Seife, eine Lotion, ein Spray, eine Creme, ein Gel, eine Emulsion, eine Reinigungsflüssigkeit oder Reinigungsmilch, ein Deodorant, ein Antitranspirant, eine Salbe, eine Haarkur oder ein Shampoo und sie kann auch in jeder der beschriebenen oder sonstigen Darreichungsformen enthalten sein, beispielsweise auch in einem Pflaster, insbesondere in einem Gel-Reservoir- oder Matrixpflaster.

In einer erfindungsgemäß besonders bevorzugten Ausführungsform handelt es sich bei der kosmetischen Zusammensetzung um ein Deodorant und/oder Antitranspirant. Das Deodorant und/oder Antitranspirant liegt hierbei vorzugsweise als Puder, in Stiftform, als Syndet, Waschlotion, Aerosolspray, Pumpspray, flüssige oder gelförmige Roll-on-Applikation, Creme, Schaum, flüssige oder feste Seife, Gel oder als getränktes flexibles Substrat vor.

Als Applikatoren können entsprechend je nach Anwendungsform beispielsweise Stifthülse, Roll-on, Pumpe, Tube, Tiegel, Spender, Tuch, Aerosoldose oder Flasche verwendet werden.

Als Applikationsort kommt die Haut jedes Körperbereichs in Frage, insbesondere die Gesichtshaut, die Kopfhaut, die Haut an den Füßen und Händen. In einer besonders bevorzugten Ausführungsform handelt es sich bei dem Applikationsort um die Haut im axillaren Bereich.

Die erfindungsgemäß verwendete kosmetische Zusammensetzung kann auch weitere Bestandteile als die zuvor genannten enthalten. In einer bevorzugten Ausführungsform enthält sie mindestens eine der im folgenden aufgezählten Substanzen. Sie kann auch jede beliebige Kombination der im folgenden aufgezählten Bestandteile enthalten.

Als erfindungsgemäß besonders vorteilhaft haben sich insbesondere kosmetische Zusammensetzungen herausgestellt, die Mischungen aus mindestens einem erfindungsgemäß verwendeten auf der Haut präbiotisch wirksamen Pflanzenextrakt und mindestens einem auf der Haut präbiotisch wirksamen Glycerinmonoalkylether enthalten.

Weiterhin haben sich als erfindungsgemäß besonders vorteilhaft kosmetische Zusammensetzungen herausgestellt, die Mischungen aus mindestens einer erfindungsgemäß verwendeten auf der Haut präbiotisch wirksamen Substanz und mindestens einem Deo-Wirkstoff, insbesondere einer antimikrobiell wirksamen Substanz, enthalten.

Bei der kombinierten Verwendung einer präbiotisch wirksamen Substanz zusammen mit einem Deo-Wirkstoff tritt hierbei vorteilhafterweise ein synergistischer Effekt auf, der darin besteht, dass die Bakterienzahl insgesamt vermindert wird, jedoch die unerwünschten Bakterien in stärkerem Maße dezimiert werden als die erwünschten Bakterien. Aufgrund dieses kombinierten Effekts sind die erwünschten Bakterien anschließend gegebenenfalls in noch stärkerem Maße dazu in der Lage, sich auf der Haut zu verbreiten, als dies bei Vorliegen eines rein präbiotischen Effektes der Fall wäre.

In einer erfindungsgemäßen Ausführungsform enthält die Zusammensetzung neben mindestens einem erfindungsgemäß verwendeten präbiotisch wirksamen Pflanzenextrakt mindestens einen weiteren Pflanzenextrakt. Dieser weitere Pflanzenextrakt kann beispielsweise durch Extraktion der gesamten Pflanze, aber auch ausschließlich durch Extraktion aus Blüten und/oder Blättern und/oder Samen und/oder anderen Pflanzenteilen, hergestellt werden. Erfindungsgemäß sind vor allem die Extrakte aus dem Meristem, also dem teilungsfähigen Bildungsgewebe der Pflanzen, und die Extrakte aus speziellen Pflanzen wie Grünem Tee, Hamamelis, Kamille, Stiefmütterchen, Paeonie, Aloe Vera, Rosskastanie, Salbei, Weidenrinde, Zimtbaum (cinnamon tree), Chrysanthemen, Eichenrinde, Brennessel, Hopfen, Klettenwurzel, Schachtelhalm, Weißdorn, Lindenblüten, Mandeln, Fichtennadeln, Sandelholz, Wacholder, Kokosnuß, Kiwi, Guave, Limette, Mango, Aprikose, Weizen, Melone, Orange, Grapefruit, Avocado, Rosmarin, Birke, Buchensprossen, Wiesenschaumkraut, Schafgarbe, Quendel, Thymian, Melisse, Hauhechel, Eibisch (Althaea), Veilchen, Blättern der Schwarzen Johannisbeere, Huflattich, Fünffingerkraut, Ginseng, Ingwerwurzel und Süßkartoffel als weiterer Pflanzenextrakt bevorzugt. Vorteilhaft eingesetzt werden können auch Algenextrakte. Die erfindungsgemäß verwendeten Algenextrakte stammen aus Grünalgen, Braunalgen, Rotalgen oder Blaualgen (Cyanobakterien). Die zur Extraktion eingesetzten Algen können sowohl natürlichen Ursprungs als auch durch biotechnologische Prozesse gewonnen und gewünschtenfalls gegenüber der natürlichen Form verändert sein. Die Veränderung der Organismen kann gentechnisch, durch Züchtung oder durch die Kultivation in mit ausgewählten Nährstoffen angereicherten Medien erfolgen. Bevorzugte Algenextrakte stammen aus Seetang, Blaualgen, aus der Grünalge Codium tomentosum sowie aus der Braunalge Fucus vesiculosus. Ein besonders bevorzugter Algenextrakt stammt aus Blaualgen der Species Spirulina, die in einem Magnesium-angereicherten Medium kultiviert wurden.

Als weiterer Pflanzenextrakt besonders bevorzugt sind die Extrakte aus Spirulina, Grünem Tee, Aloe Vera, Meristem, Harnamelis, Aprikose, Guave, Süßkartoffel, Limette, Mango, Kiwi, Gurke, Malve, Eibisch und Veilchen. Die erfindungsgemäßen Mittel können als zusätzlichen Pflanzenextrakt auch Mischungen aus mehreren, insbesondere aus zwei, verschiedenen Pflanzenextrakten enthalten.

Als Extraktionsmittel zur Herstellung der genannten weiteren Pflanzenextrakte können ebenso wie zur Herstellung der präbiotisch wirksamen Pflanzenextrakte beispielsweise Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol, Propylenglykol und Butylenglykol und zwar sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen. Die Wasserdampfdestillation fällt erfindungsgemäß unter die bevorzugten Extraktionsverfahren. Die Extraktion kann aber gegebenenfalls auch in Form von Trockenextraktion erfolgen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch. Je nach Wahl der Extraktionsmittel kann es bevorzugt sein, den Pflanzenextrakt durch Zugabe eines Lösungsvermittlers zu stabilisieren. Als Lösungsvermittler geeignet sind z. B. Ethoxylierungsprodukte von gegebenenfalls gehärteten pflanzlichen und tierischen Ölen. Bevorzugte Lösungsvermittler sind ethoxylierte Mono-, Di- und Triglyceride von C₈₋₂₂-Fettsäuren mit 4 bis 50 Ethylenoxid-Einheiten, z. B. hydriertes ethoxyliertes Castoröl, Olivenölethoxylat, Mandelölethoxylat, Nerzölethoxylat, Polyoxyethylenglykolcapryl-/-/caprinsäureglyceride, Polyoxyethylenglycerinmonolaurat und Polyoxyethylenglykolkokosfettsäureglyceride.

Weiterhin kann es bevorzugt sein, in den erfindungsgemäß verwendeten Mitteln Mischungen aus mehreren, insbesondere aus zwei, verschiedenen Pflanzenextrakten zusätzlich zu dem präbiotisch wirksamen Pflanzenextrakt einzusetzen.

Hinsichtlich der erfindungsgemäß verwendbaren Pflanzenextrakte wird weiterhin auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Die kosmetischen Zusammensetzungen und insbesondere die erfindungsgemäß bevorzugten Deodorant- oder Antitranspirant-Zusammensetzungen, die die erfindungsgemäß verwendeten präbiotisch aktiven Substanzen enthalten, können weiterhin Fettstoffe enthalten. Unter Fettstoffen sind Fettsäuren, Fettalkohole, natürliche und synthetische kosmetische Ölkomponenten sowie natürliche und synthetische Wachse zu verstehen, die sowohl in fester Form als auch flüssig in wässriger oder öliger Dispersion vorliegen können.

Als Fettsäuren können eingesetzt werden lineare und/oder verzweigte, gesättigte und/ oder ungesättigte C₈₋₃₀-Fettsäuren. Bevorzugt sind C₁₀₋₂₂-Fettsäuren. Beispiele sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachidonsäure, Gadoleinsäure, Behensäure und Erucasäure sowie deren technische Mischungen. Besonders bevorzugt ist der Einsatz von Stearinsäure Die eingesetzten Fettsäuren können eine oder mehrere Hydroxygruppen tragen. Bevorzugte Beispiele hierfür sind die α-Hydroxy-C₈-C₁₈-Carbonsäuren sowie 12-Hydroxystearinsäure. Die Einsatzmenge beträgt dabei 0,1 - 15 Gew.-%, bevorzugt 0,5 - 10 Gew.-%, besonders bevorzugt 1 - 5 Gew.%, jeweils bezogen auf die gesamte Zusammensetzung.

Als Fettalkohole können eingesetzt werden gesättigte, ein- oder mehrfach ungesättigte, verzweigte oder unverzweigte Fettalkohole mit 6 - 30, bevorzugt 10 - 22 und ganz besonders bevorzugt 12-22 Kohlenstoffatomen. Einsetzbar im Sinne der Erfindung sind z.B. Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole.

Für Stiftformulierungen werden häufig Wachse verwendet. Als natürliche oder synthetische Wachse können erfindungsgemäß eingesetzt werden feste Paraffine oder Isoparaffine, Pflanzenwachse wie Candelillawachs, Carnaubawachs, Espartograswachs, Japanwachs, Korkwachs, Zuckerrohrwachs, Ouricurywachs, Montanwachs, Sonnenblumenwachs, Fruchtwachse und tierische Wachse, wie z. B. Bienenwachse und andere Insektenwachse, Walrat, Schellackwachs, Wollwachs und Bürzelfett, weiterhin Mineralwachse, wie z. B. Ceresin und Ozokerit oder die petrochemischen Wachse, wie z. B. Petrolatum, Paraffinwachse, Microwachse aus Polyethylen oder Polypropylen und Polyethylenglycolwachse. Es kann vorteilhaft sein, hydrierte oder gehärtete Wachse einzusetzen. Weiterhin sind auch chemisch modifizierte Wachse, insbesondere die Hartwachse, z. B. Montanesterwachse, Sasolwachse und hydrierte Jojobawachse, einsetzbar.

Weiterhin geeignet sind die Mono-, Di- und Triglyceride gesättigter und gegebenenfalls hydroxylierter C₁₆₋₃₀-Fettsäuren, wie z. B. gehärtete Triglyceridfette (hydriertes Palmöl, hydriertes Kokosöl, hydriertes Rizinusöl), Glycerylmonostearat (Cutina^{®} MD), Glyceryltribehenat oder Glyceryltri-12-hydroxystearat, weiterhin synthetische Vollester aus Fettsäuren und Glykolen (z. B. Syncrowachs^{®}) oder Polyolen mit 2-6 C-Atomen, Fettsäuremonoalkanolamide mit einem C₁₂₋₂₂-Acylrest und einem C₂₋₄-Alkanolrest, Ester aus gesättigten und/oder ungesättigten, verzweigten und/oder unverzweigten Alkancarbonsäuren einer Kettenlänge von 1 bis 80 C-Atomen und gesättigten und/oder ungesättigten, verzweigten und/oder unverzweigten Alkoholen einer Kettenlänge von 1 bis 80 C-Atomen, darunter z. B. synthetische Fettsäure-Fettalkoholester wie Stearylstearat oder Cetylpalmitat, Ester aus aromatischen Carbonsäuren, Dicarbonsäuren bzw. Hydroxycarbonsäuren (z. B. 12-Hydroxystearinsäure) und gesättigten und/oder ungesättigten, verzweigten und/ oder unverzweigten Alkoholen einer Kettenlänge von 1 bis 80 C-Atomen, Lactide langkettiger Hydroxycarbonsäuren und Vollester aus Fettalkoholen und Di- und Tricarbonsäuren, z. B. Dicetylsuccinat oder Dicetyl-/stearyladipat, sowie Mischungen dieser Substanzen, sofern die einzelnen Wachskomponenten oder ihre Mischung bei Raumtemperatur fest sind.

Besonders bevorzugt ist, die Wachskomponenten zu wählen aus der Gruppe der Ester aus gesättigten, unverzweigten Alkancarbonsäuren einer Kettenlänge von 14 bis 44 C-Atomen und gesättigten, unverzweigten Alkoholen einer Kettenlänge von 14 bis 44 C-Atomen, sofern die Wachskomponente oder die Gesamtheit der Wachskomponenten bei Raumtemperatur fest sind. Insbesondere vorteilhaft können die Wachskomponenten aus der Gruppe der C₁₆₋₃₆-Alkylstearate, der C₁₀₋₄₀-Alkylstearate, der C₂₋₄₀-Alkylisostearate, der C₂₀₋₄₀-Dialkylester von Dimersäuren, der C₁₈₋₃₈-Alkylhydroxystearoylstearate, der C₂₀₋₄₀-Alkylerucate gewählt werden, ferner sind C₃₀₋₅₀-Alkylbienenwachs sowie Cetearylbehenat einsetzbar. Auch Silikonwachse, zum Beispiel Stearyltrimethylsilan/Stearylalkohol sind gegebenenfalls vorteilhaft. Besonders bevorzugte Wachskomponenten sind die Ester aus gesättigten, einwertigen C₂₀-C₆₀-Alkoholen und gesättigten C₈-C₃₀-Moriocarbonsäuren, insbesondere ein C₂₀-C₄₀-Alkylstearat bevorzugt, das unter dem Namen Kesterwachs^{®} K82H von der Firma Koster Keunen Inc. erhältlich ist. Das Wachs oder die Wachskomponenten sollten bei 25° C fest sein, jedoch im Bereich von 35 - 95°C schmelzen, wobei ein Bereich von 45 - 85 °C bevorzugt ist.

Natürliche, chemisch modifizierte und synthetische Wachse können alleine oder in Kombination eingesetzt werden.

Die Wachskomponenten sind in einer Menge von 0,1 bis 40 Gew.-%, bezogen auf die Gesamtzusammensetzung, vorzugsweise 1 bis 30 Gew.-% und insbesondere 5 - 15 Gew.-% enthalten.

Die erfindungsgemäß verwendeten Zusammensetzungen können weiterhin wenigstens ein unpolares oder polares flüssiges Öl, das natürlich oder synthetisch sein kann, enthalten. Die polare Ölkomponente kann ausgewählt sein aus pflanzlichen Ölen, z. B. Sonnenblumenöl, Olivenöl, Sojaöl, Rapsöl, Mandelöl, Jojobaöl und den flüssigen Anteilen des Kokosöls sowie synthetischen Triglyceridölen, aus Esterölen, das heißt den Estern von C₆₋₃₀-Fettsäuren mit C₂₋₃₀-Fettalkohalen, aus Dicarbonsäureestern wie Di-n-butyladipat, Di-(2-ethylhexyl)-adipat und Di-(2-ethylhexyl)-succinat sowie Diolestern wie Ethylenglykoldioleat und Propylenglykoldi(2-ethylhexanoat), aus symmetrischen, unsymmetrischen oder cyclischen Estern der Kohlensäure mit Fettalkoholen, beispielsweise beschrieben in der DE-OS 197 56 454, Glycerincarbonat oder Dicaprylylcarbonat (Cetiol^{®} CC), aus Mono,- Di- und Trifettsäureestern von gesättigten und/oder ungesättigten linearen und/oder verzweigten Fettsäuren mit Glycerin, aus verzweigten Alkanolen, z. B. Guerbet-Alkoholen mit einer einzigen Verzweigung am Kohlenstoffatom 2 wie 2-Hexyldecanol, 2-Octyldodecanol, Isotridecanol und Isohexadecanol, aus Alkandiolen, z. B. den aus Epoxyalkanen mit 12 - 24 C-Atomen durch Ringöffnung mit Wasser erhältlichen vicinalen Diolen, aus Etheralkoholen, z. B. den Monoalkylethern des Glycerins, des Ethylenglycols, des 1,2-Propylenglycols oder des 1,2-Butandiols, aus Dialkylethern mit jeweils 12 - 24 C-Atomen, z. B. den Alkyl-methylethern oder Di-n-alkylethern mit jeweils insgesamt 12 - 24 C-Atomen, insbesondere Di-n-octylether (Cetiol^{®}OE ex Cognis), sowie aus Anlagerungsprodukten von Ethylenoxid und/oder Propylenoxid an ein- oder mehrwertige C₃₋₂₀-Alkanole wie Butanol und Glycerin, z. B. PPG-3-Myristylether (Witconol^{®} APM), PPG-14-Butylether (Ucon Fluid^{®} AP), PPG-15-Stearylether (Arlamol^{®} E), PPG-9-Butylether (Breox^{®} B25) und PPG-10-Butandiol (Macol^{®} 57). Die unpolare Ölkomponente kann ausgewählt sein aus flüssigen Paraffinölen, Isoparaffinölen, z. B. Isohexadecan und Isoeicosan, aus hydrogenierten Polyalkenen, insbesondere Poly-1-decenen (im Handel erhätlich als Nexbase 2004, 2006 oder 2008 FG (Fortum, Belgien)), aus synthetischen Kohlenwasserstoffen, z. B. 1,3-Di-(2-ethyl-hexyl)-cyclohexan (Cetiol^{®} S), sowie aus flüchtigen und nichtflüchtigen Silikonölen, die cyclisch, wie z. B. Decamethylcyclopentasiloxan und Dodecamethylcyclohexasiloxan, oder linear sein können, z. B. lineares Dimethylpolysiloxan, im Handel erhältlich z. B. unter der Bezeichnung Dow Corning^{®} 190, 200, 244, 245, 344 oder 345 und Baysilon^{®} 350 M.

Die erfindungsgemäß verwendeten Zusammensetzungen können weiterhin wenigstens einen wasserlöslichen Alkohol enthalten. Unter Wasserlöslichkeit versteht man erfindungsgemäß, dass sich wenigstens 5 Gew.-% des Alkohols bei 20 °C klar lösen oder aber - im Falle langkettiger oder polymerer Alkohole - durch Erwärmen der Lösung auf 50 °C bis 60 °C in Lösung gebracht werden können. Geeignet sind je nach Darreichungsform einwertige Alkohole wie z. B. Ethanol, Propanol oder Isopropanol. Weiterhin geeignet sind wasserlösliche Polyole. Hierzu zählen wasserlösliche Diole,' Triole und höherwertige Alkohole sowie Polyethylenglycole. Unter den Diolen eignen sich C₂-C₁₂-Diole, insbesondere 1,2-Propylenglycol, Butylenglycole wie z. B. 1,2-Butylenglycol, 1,3-Butylenglycol und 1,4-Butylenglycol, Hexandiole wie z. B. 1,6-Hexandiol. Weiterhin bevorzugt geeignet sind Glycerin und insbesondere Diglycerin und Triglycerin, 1,2,6-Hexantriol sowie die Dipropylenglycol und die Polyethylenglycole (PEG) PEG-400, PEG-600, PEG-1000, PEG-1550, PEG-3000 und PEG-4000.

Die Menge des Alkohols oder des Alkohol-Gemisches in den erfindungsgemäß verwendeten Zusammensetzungen beträgt 1 - 50 oder 1 - 70 Gew.-% und vorzugsweise 5 - 40 oder 5 - 55 Gew.-%, bezogen auf die gesamte Zusammensetzung. Erfindungsgemäß kann sowohl ein Alkohol als auch ein Gemisch mehrerer Alkohole eingesetzt werden.

Die erfindungsgemäß verwendeten Zusammensetzungen können im wesentlichen wasserfrei sein, das heißt maximal 5 Gew.-%, bevorzugt maximal 1 Gew.-% Wasser enthalten. In wasserhaltigen Darreichungsformen beträgt der Wassergehalt 5 - 98 Gew.-%, bevorzugt 10 - 90 und besonders bevorzugt 15 - 85 Gew.-%, bezogen auf die gesamte Zusammensetzung.

Die erfindungsgemäß verwendeten Zusammensetzungen können weiterhin Emulgatoren und/oder Tenside enthalten. In einer besonders bevorzugten Ausführungsform handelte sich hierbei um Anlagerungsprodukte von 10 - 40 Mol Ethylenoxid an lineare oder verzweigte Fettalkohole mit 16 - 22 C-Atomen, an Fettsäuren mit 12 - 22 C-Atomen, an Fettsäurealkanolamide, an Fettsäuremonoglyceride, an Sorbitan-Fettsäuremonoester, an Fettsäurealkanolamide, an Fettsäureglyceride, z.B. an gehärtetes Rizinusöl, an Methylglucosidmonofettsäureester und Gemische davon. Grundsätzlich können jedoch auch beliebige andere Emulgatoren und/oder Tenside verwendet werden. Die erfindungsgemäßen Mittel enthalten die Emulgatoren bevorzugt in Mengen von 0,1 bis 25 Gew.-%, insbesondere 0,5 - 15 Gew.-%, bezogen auf das gesamte Mittel.

Weiterhin können die erfindungsgemäß verwendeten Zusammensetzungen mindestens ein Proteinhydrolysat oder dessen Derivat enthalten. Erfindungsgemäß können sowohl pflanzliche als auch tierische Proteinhydrolysate eingesetzt werden. Tierische Proteinhydrolysate sind z. B. Elastin-, Collagen-, Keratin-, Seiden- und Milcheiweiß-Proteinhydrolysate, die auch in Form von Salzen vorliegen können. Erfindungsgemäß bevorzugt sind pflanzliche Proteinhydrolysate, z. B. Soja-, Weizen-, Mandel-, Erbsen-, Kartoffel- und Reisproteinhydrolysate. Entsprechende Handelsprodukte sind z. B. DiaMin^{®} (Diamalt), Gluadin^{®} (Cognis), Lexein^{®} (Inolex) und Crotein^{®} (Croda).

An Stelle der Proteinhydrolysate können zum einen anderweitig erhaltene Aminosäuregemische, zum anderen auch einzelne Aminosäuren sowie deren physiologisch verträgliche Salze eingesetzt werden. Zu den erfindungsgemäß bevorzugten Aminosäuren gehören Glycin, Serin, Threonin, Cystein, Asparagin, Glutamin, Pyroglutaminsäure, Alanin, Valin, Leucin, Isoleucin, Prolin, Tryptophan, Phenylalanin, Methionin, Asparaginsäure, Glutaminsäure, Lysin, Arginin und Histidin sowie die Zinksalze und die Säureadditionssalze der genannten Aminosäuren.

Ebenfalls möglich ist der Einsatz von Derivaten der Proteinhydrolysate, z. B. in Form ihrer Fettsäure-Kondensationsprodukte. Entsprechende Handelsprodukte sind z. B. Lamepon^{®} (Cognis), Gluadin^{®} (Cognis), Lexein^{®} (Inoiex), Crolastin^{®} oder Crotein^{®} (Croda).

Erfindungsgemäß einsetzbar sind auch kationisierte Proteinhydrolysate, wobei das zugrunde liegende Proteinhydrolysat vom Tier, von der Pflanze, von marinen Lebensformen öder von biotechnologisch gewonnenen Proteinhydrolysaten, stammen kann. Bevorzugt sind kationische Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Weiterhin können die kationischen Proteinhydrolysate auch noch weiter derivatisiert sein. Als typische Beispiele für erfindungsgemäß verwendete kationische Proteinhydrolysate und derivate seien einige der unter den INCI - Bezeichnungen im "International Cosmetic Ingredient Dictionary and Handbook", (seventh edition 1997, The Cosmetic, Toiletry, and Fragrance Association 1101 17th Street, N.W., Suite 300, Washington, DC 20036-4702) genannten und im Handel erhältlichen Produkte aufgeführt: Cocodimonium Hydroxypropyl Hydrolyzed Collagen, Cocodimonium Hydroxypropyl Hydrolyzed Casein, Steardimonium Hydroxypropyl Hydrolyzed Collagen, Steardimonium Hydroxypropyl Hydrolyzed Hair Keratin, Lauryldimonium Hydroxypropyl Hydrolyzed Keratin, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCl, Hydroxypropyltrimonium Gelatin. Ganz besonders bevorzugt sind die kationischen Proteinhydrolysate und -derivate auf pflanzlicher Basis.

In den erfindungsgemäß verwendeten Zusammensetzungen sind die Proteinhydrolysate und deren Derivate beziehungsweise die Aminosäuren und deren Derivate in Mengen bis zu 10 Gew.-%, bezogen auf das gesamte Mittel, enthalten. Mengen von 0,1 bis 5 Gew.%, insbesondere 0,1 bis 3 Gew.%, sind besonders bevorzugt.

Weiterhin können die erfindungsgemäß verwendeten Zusammensetzungen mindestens ein Mono-, Oligo- oder Polysaccharid oder deren Derivate enthalten.

Erfindungsgemäß geeignete Monosaccharide sind z. B. Glucose, Fructose, Galactose, Arabinose, Ribose, Xylose, Lyxose, Allose, Altrose, Mannose, Gulose, Idose und Talose, die Desoxyzucker Fucose und Rhamnose sowie Aminozucker wie z. B. Glucosamin oder Galactosamin. Bevorzugt sind Glucose, Fructose, Galactose, Arabinose und Fucose; Glucose ist besonders bevorzugt.

Erfindungsgemäß geeignete Oligosaccharide sind aus zwei bis zehn Monosaccharideinheiten zusammengesetzt, z. B. Saccharose, Lactose oder Trehalose. Ein besonders bevorzugtes Oligosaccharid ist Saccharose. Ebenfalls besonders bevorzugt ist die Verwendung von Honig, der überwiegend Glucose und Saccharose enthält.

Erfindungsgemäß geeignete Polysaccharide sind aus mehr als zehn Monosaccharideinheiten zusammengesetzt. Bevorzugte Polysaccharide sind die aus α-D-Glucose-Einheiten aufgebauten Stärken sowie Stärkeabbauprodukte wie Amylose, Amylopektin und Dextrine. Erfindungsgemäß besonders vorteilhaft sind chemisch und/oder thermisch modifizierte Stärken, z. B. Hydroxypropylstärkephosphat, Dihydroxypropyldistärkephosphat oder die Handelsprodukte Dry Flo^{®}. Weiterhin bevorzugt sind Dextrane sowie ihre Derivate, z. B. Dextransulfat. Ebenfalls bevorzugt sind nichtionische Cellulose-Derivate, wie Methylcellulose, Hydroxypropylcellulose oder Hydroxyethylcellulose, sowie kationische Cellulose-Derivate, z. B. die Handelsprodukte Celquat^{®} und Polymer JR^{®}, und bevorzugt Celquat^{®} H 100, Celquat^{®} L 200 und Polymer JR^{®} 400 (Polyquaternium-10) sowie Polyquaternium-24. Weitere bevorzugte Beispiele sind Polysaccharide aus Fucose-Einheiten, z. B. das Handelsprodukt Fucogel^{®}. Besonders bevorzugt sind die aus Aminozuckereinheiten aufgebauten Polysaccharide, insbesondere Chitine und ihre deacetylierten Derivate, die Chitosane, und Mucopolysaccharide. Zu den erfindungsgemäß bevorzugten Mucopolysacchariden gehören Hyaluronsäure und ihre Derivate, z. B. Natriumhyaluronat oder Dimethylsilanolhyaluronat, sowie Chondroitin und seine Derivate, z. B. Chondroitinsulfat.

In einer vorteilhaften Ausführungsform enthalten die erfindungsgemäßen Zusammensetzungen mindestens ein filmbildendes, emulsionsstabilisierendes, verdickendes oder adhäsives Polymer, ausgewählt aus natürlichen und synthetischen Polymeren, die kationisch, anionisch, amphoter geladen oder nichtionisch sein können.

Erfindungsgemäß bevorzugt sind kationische, anionische sowie nichtionische Polymere.

Unter den kationischen Polymeren bevorzugt sind Polysiloxane mit quaternären Gruppen, z. B. die Handelsprodukte Q2-7224 (Dow Corning), Dow Corrling^{®} 929 Emulsion (mit Amodimethicone), SM-2059 (General Electric), SLM-55067 (Wacker) sowie Abil^{®}-Quat 3270 und 3272 (Th. Goldschmidt).

Bevorzugte anionische Polymere, die die Wirkung des erfindungsgemäß verwendeten Wirkstoffs unterstützen können, enthalten Carboxylat- und/oder Sulfonatgruppen und als Monomere zum Beispiel Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure. Ganz besonders bevorzugte anionische Polymere enthalten als alleiniges Monomer oder als Comonomer 2-Acrylamido-2-methylpropansulfonsäure, wobei die Sulfonsäuregruppe ganz oder teilweise in Salzform vorliegen kann. Innerhalb dieser Ausführungsform ist es bevorzugt, Copolymere aus mindestens einem anionischen Monomer und mindestens einem nichtionischen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester. Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol% 2-Acrylamido-2-methylpropansulfonsäure, wobei die Sulfonsäuregruppen ganz oder teilweise als Natrium-, Kalium-, Ammonium-, Mono- oder Triethanolammonium-Salz vorliegen. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel^{®}305 der Firma SEPPIC enthalten. Die Verwendung dieses Compounds hat sich im Rahmen der erfindungsgemäßen Lehre als besonders vorteilhaft erwiesen. Auch die unter der Bezeichnung Simulgel^{®}600 als Compound mit Isohexadecan und Polysorbat-80 vertriebenen Natriumacryloyldimethyltaurat-Copolymere haben sich als erfindungsgemäß besonders wirksam erwiesen.

Weitere bevorzugte anionische Homo- und Copolymere sind unvernetzte und vernetzte Polyacrylsäuren. Dabei können Allylether von Pentaerythrit, von Sucrose und von Propylen bevorzugte Vernetzungsagentien sein. Solche Verbindungen sind zum Beispiel die Handelsprodukte Carbopol^{®}. Ein besonders bevorzugtes anionisches Copolymer enthält als Monomer zu 80 - 98 % eine ungesättigte, gewünschtenfalls substituierte C₃₋₆-Carbonsäure oder ihr Anhydrid sowie zu 2 - 20 % gewünschtenfalls substituierte Acrylsäureester von gesättigten C₁₀₋₃₀-Carbonsäuren, wobei das Copolymer mit den vorgenannten Vernetzungsagentien vernetzt sein kann. Entsprechende Handelsprodukte sind Pemulen^{®} und die Carbopol^{®}-Typen 954, 980, 1342 und ETD 2020 (ex B.F. Goodrich).

Geeignete nichtionische Polymere sind beispielsweise Polyvinylalkohole, die teilverseift sein können, z. B. die Handelsprodukte Mowiol^{®} sowie VinylpyrrolidonNinylester-Copolymere und Polyvinylpyrrolidone, die z. B. unter dem Warenzeichen Luviskol^{®} (BASF) vertrieben werden.

Die erfindungsgemäß verwendeten Zusammensetzungen können weiterhin mindestens eine α-Hydroxycarbonsäure oder α-Ketocarbonsäure oder deren Ester-, Lacton- oder Salzform enthalten. Geeignete α-Hydroxycarbonsäuren oder a-Ketocarbonsäuren sind ausgewählt aus Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Mandelsäure, 4-Hydroxymandelsäure, Apfelsäure, Erythrarsäure, Threarsäure, Glucarsäure, Galactarsäure, Mannarsäure, Gularsäure, 2-Hydroxy-2-methylbernsteinsäure, Gluconsäure, Brenztraubensäure, Glucuronsäure und Galacturonsäure. Die Ester der genannten Säuren sind ausgewählt aus den Methyl-, Ethyl-, Propyl-, Isopropyl-, Butyl-, Amyl-, Pentyl-, Hexyl-, 2-Ethylhexyl-, Octyl-, Decyl-, Dodecyl- und Hexadecylestern. Die α-Hydroxycarbonsäuren oder α-Ketocarbonsäuren oder ihre Derivate sind in Mengen von 0,1 - 10 Gew.-%, bevorzugt 0,5 - 5 Gew.-%, jeweils bezogen auf die gesamte Zusammensetzung, enthalten.

Die erfindungsgemäß verwendeten Zusammensetzungen enthalten in einer bevorzugten Ausführungsform mindestens einen Antitranspirant-Wirkstoff. Als Antitranspirant-Wirkstoffe eignen sich erfindungsgemäß wasserlösliche adstringierende oder einweißkoagulierende metallische Salze, insbesondere anorganische und organische Salze des Aluminiums, Zirkoniums, Zinks und Titans sowie beliebige Mischungen dieser Salze. Erfindungsgemäß wird unter Wasserlöslichkeit eine Löslichkeit von wenigstens 4 g Aktivsubstanz pro 100 g Lösung bei 20 °C verstanden. Erfindungsgemäß verwendbar sind beispielsweise Alaun (KAl(SO₄)₂. 12 H₂O), Aluminiumsulfat, Aluminiumlactat, Natrium-Aluminium-Chlorhydroxylactat, Aluminiumchlorhydroxyallantoinat, Aluminiumchlorohydrat, Aluminiumsulfocarbolat, Aluminium-Zirkonium-Chlorohydrat, Zinkchlorid, Zinksulfocarbolat, Zinksulfat, Zirkoniumchlorohydrat, Aluminium-Zirkonium-Chlorohydrat-Glycin-Komplexe und Komplexe von basischen Aluminiumchloriden mit Propylenglycol oder Polyethylenglycol. Bevorzugt enthalten die flüssigen Wirkstoffzubereitungen ein adstringierendes Aluminiumsalz, insbesondere Aluminiumchlorohydrat, und/ oder eine Aluminium-Zirkonium-Verbindung. Aluminiumchlorohydrate werden beispielsweise pulverförmig als Micro Dry^{®} Ultrafine oder in aktivierter Form als Reach^{®} 501 oder Reach^{®} 103 von Reheis sowie in Form wäßriger Lösungen als Locron^{®} L von Clariant oder als Chlorhydrol^{®} von Reheis vertrieben. Unter der Bezeichnung Reach^{®} 301 wird ein Aluminiumsesquichlorohydrat von Reheis angeboten. Auch die Verwendung von Aluminium-Zirkonium-Tri- oder Tetrachlorohydrex-Glycin-Komplexen, die beispielsweise von Reheis unter der Bezeichnung Rezal^{®} 36G im Handel sind, ist erfindungsgemäß besonders vorteilhaft.

Der schweißhemmende Wirkstoff ist in den erfindungsgemäß verwendeten Zusammensetzungen in einer Menge von 0,01 - 40 Gew.-%, vorzugsweise 2 - 30 Gew.-% und insbesondere 5 - 25 Gew.-%, bezogen auf die Menge der Aktivsubstanz in der gesamten Zusammensetzung, enthalten.

Die erfindungsgemäß verwendeten Zusammensetzungen enthalten in einer weiteren besonders bevorzugten Ausführungsform neben der präbiotisch aktiven Substanz mindestens einen weiteren Deodorant-Wirkstoff. Erfindungsgemäß als weitere Deodorant-Wirkstoffe geeignet sind Duftstoffe, antimikrobielle, antibakterielle oder keimhemmende Stoffe, enzymhemmende Stoffe, Antioxidantien und Geruchsadsorbentien.

Geeignet sind insbesondere Organohalogenverbindungen sowie -halogenide, quartäre Ammoniumverbindungen und Zinkverbindungen. Bevorzugt sind Chlorhexidin und Chlorhexidingluconat, Benzalkoniumhalogenide und Cetylpyridiniumchlorid. Desweiteren sind Natriumbicarbonat, Natriumphenolsulfonat und Zinkphenolsulfonat, die Bestandteile des Lindenblütenöls und des Kamillenöls, Bisabolol, Phenoxyethanol, Triclosan (Irgasan® DP300) oder Triethylcitrat einsetzbar.

Als Enzym-hemmende Stoffe bevorzugt sind Inhibitoren für Enzyme der axillaren Keimflora, die bei der Entstehung von Körpergeruch involviert sind. Hierbei handelt es sich vorzugsweise um Inhibitoren von Lipasen, Arylsulfatasen (s. WO 01/99376), β-Glucoronidasen (s. WO 03/039505), 5-α-Reduktasen und Aminoacylasen.

Weitere antibakteriell wirksame Deodorant-Wirkstoffe sind Lantibiotika, Glycoglycerolipide, Sphingolipide (Ceramide), Sterine und andere Wirkstoffe, die die Bakterienadhäsion an der Haut inhibieren, z. B. Glycosidasen, Lipasen, Proteasen, Kohlenhydrate, Di- und Oligosaccharidfettsäureester sowie alkylierte Mono- und Oligosaccharide.

Weiterhin geeignet als Deodorant-Wirkstoff sind wasserlösliche Polyole, ausgewählt aus wasserlöslichen Diolen, Triolen und höherwertigen Alkoholen sowie Polyethylenglycolen. Unter den Diolen eignen sich C₂-C₁₂-Diole, insbesondere 1,2-Propylenglycol, Butylenglycole wie z. B. 1,2-Butylenglycol, 1,3-Butylenglycol und 1,4-Butylenglycol, Pentandiole, z. B. 1,2-Pentandiol, sowie Hexandiole, z. B. 1,6-Hexandiol. Weiterhin bevorzugt geeignet sind Glycerin und technische Oligoglyceringemische mit einem Eigenkondensationsgrad von 1,5 bis 10 wie etwa technische Diglyceringemische mit einem Diglyceringehalt von 40 bis 50 Gew.-% oder Triglycerin, weiterhin 1,2,6-Hexantriol sowie Polyethylenglycole (PEG) mit einem durchschnittlichen Molekulargewicht von 100 bis 1.000 Dalton, beispielsweise PEG-400, PEG-600 oder PEG-1000. Weitere geeignete höherwertige Alkohole sind die C₄-, C₅- und C₆-Monosaccharide und die entsprechenden Zuckeralkohole, z. B. Mannit oder Sorbit.

Deodorant- oder Antitranspirant-Stifte können in gelierter Form, auf wasserfreier Wachsbasis und auf Basis von W/O-Emulsionen und O/W-Emulsionen vorliegen. Gelstifte können auf der Basis von Fettsäureseifen, Dibenzylidensorbitol, N-Acylaminosäureamiden, 12-Hydroxystearinsäure und anderen Gelbildnern hergestellt werden. Aerosolsprays, Pumpsprays, Roll on-Applikationen und Cremes können als Wasser-in-Öl-Emulsion, Öl-in-Wasser-Emulsion, Siliconöl-in-Wasser-Emulsion, Wasser-in-Öl-Mikroemulsion, Öl-in-Wasser-Mikroemulsion, Siliconöl-in-Wasser-Mikroemulsion, wasserfreie Suspension, alkoholische und hydroalkoholische Lösung, wässriges Gel und als Öl vorliegen. Alle genannten Zusammensetzungen können verdickt sein, beispielsweise auf der Basis von Fettsäureseifen, Dibenzylidensorbitol, N-Acylaminosäureamiden, 12-Hydroxystearinsäure, Polyacrylaten vom Carbomer- und Carbopol-Typ, Polyacrylamiden und Polysacchariden, die chemisch und/oder physikalisch modifiziert sein können. Die Emulsionen und Mikroemulsionen können transparent, translucent oder opak sein.

Flüssige und gelförmige Darreichungsformen der erfindungsgemäß verwendeten Zusammensetzungen können Verdickungsmittel enthalten, z. B. Celluloseether, wie Hydroxypropylcellulose, Hydroxyethylcellulose und Methylhydroxypropylcellulose, verdickende Polymere auf Basis von Polyacrylaten, die gewünschtenfalls vernetzt sein können, z. B. die Carbopoltypen oder Pemulen^{®}-Produkte, oder auf Basis von Polyacrylamiden oder sulfonsäuregruppenhaltigen Polyacrylaten, z. B Sepigel^{®} 305 oder Simulgel^{®} EG, weiterhin anorganische Verdicker, z. B. Bentonite und Hectorite (Laponite^{®}).

Die erfindungsgemäß verwendeten Zusammensetzungen können weitere kosmetisch und dermatologisch wirksame Stoffe enthalten, wie beispielsweise entzündungshemmende Substanzen, Feststoffe, ausgewählt aus Kieselsäuren, z. B. Aerosil^{®}-Typen, Kieselgelen, Siliciumdioxid, Tonen, z. B. Bentonite oder Kaolin, Magnesiumaluminiumsilikaten, z.B. Talkum, Bornitrid, Titandioxid, das gewünschtenfalls beschichtet sein kann, gegebenenfalls modifizierten Stärken und Stärkederivaten, Cellulosepulvern und Polymerpulvern, desweiteren Pflanzenextrakte, Proteinhydrolysate, Vitamine, Parfümöle, Sebostatika, Anti-Akne-Wirkstoffe sowie Keratolytika.

Die erfindungsgemäß verwendeten Zusammensetzungen können, soweit sie flüssig vorliegen, auf flexible und saugfähige Träger aufgebracht und als Deodorant- oder Antitranspirant-Tücher oder Schwämmchen angeboten werden. Als flexible und saugfähige Träger im Sinne der Erfindung eignen sich z. B. Träger aus Textilfasern, Kollagen oder polymeren Schaumstoffen. Als Textilfasern können sowohl Naturfasern wie Cellulose (Baumwolle, Leinen), Seide, Wolle, Regeneratcellulose (Viskose, Rayon), Cellulosederivate als auch synthetische Fasern wie z.B. Polyester, Polyacrylnitril, Polyamid- oder Polyolefinfasern oder Mischungen solcher Fasern gewebt oder ungewebt verwendet werden. Diese Fasern können zu saugfähigen Wattepads, Vliesstoffen oder zu Geweben oder Gewirken verarbeitet sein. Auch flexible und saugfähige polymere Schaumstoffe, z. B. Polyurethanschäume und Polyamidschäume sind geeignete Substrate. Das Substrat kann eine, zwei, drei sowie mehr als drei Lagen aufweisen, wobei die einzelnen Lagen aus gleichen oder unterschiedlichen Materialien bestehen können. Jede Substratschicht kann eine homogene oder eine inhomogene Struktur mit beispielsweise verschiedenen Zonen unterschiedlicher Dichte aufweisen.

Als saugfähig im Sinne der Erfindung sind solche Trägersubstrate anzusehen, die bei 20° C wenigstens 10 Gew.-%, bezogen auf das Trockengewicht, an Wasser adsorptiv bzw. kapillar binden können. Bevorzugt eignen sich aber solche Träger, die wenigstens 100 Gew.-% Wasser adsorptiv und kapillar binden können.

Die Ausrüstung der Trägersubstrate erfolgt in der Weise, daß man die saugfähigen, flexiblen Trägersubstrate, bevorzugt aus Textilfasern, Kollagen oder polymeren Schaumstoffen mit den erfindungsgemäßen Zusammensetzungen behandelt bzw. ausrüstet und gegebenenfalls trocknet. Dabei kann die Behandlung (Ausrüstung) der Trägersubstrate nach beliebigen Verfahren, z. B. durch Aufsprühen, Tauchen und Abquetschen, Durchtränken oder einfach durch Einspritzen der erfindungsgemäßen Zusammensetzung in die Trägersubstrate erfolgen.

Erfindungsgemäß bevorzugt ist weiterhin die Darreichungsform als Aerosol, wobei die kosmetische Zusammensetzung ein Treibmittel, vorzugsweise ausgewählt aus Propan, Butan, Isobutan, Pentan, Isopentan, Dimethylether, Fluorkohlenwasserstoffen und Fluorchlorkohlenwasserstoffen enthält. Ebenso kann ein komprimiertes Treibmittel wie Luft, Stickstoff oder Kohlendioxid verwendet werden. Ebenso können Mischungen der genannten Treibmittel eingesetzt werden.

In einer bevorzugten Ausführungsform liegen die erfindungsgemäß verwendeten Zusammensetzungen in Form einer flüssigen oder festen Öl-in-Wasser-Emulsion, Wasser-in-Öl-Emulsion, Mehrfach-Emulsion, Mikroemulsion, PIT-Emulsion oder Pickering-Emulsion, eines Hydrogels, eines Lipogels, einer ein- oder mehrphasigen Lösung, eines Schaumes, eines Puders oder einer Mischung mit mindestens einem als medizinischen Klebstoff geeigneten Polymer vor. Die Mittel können auch in wasserfreier Form, wie beispielsweise einem Öl oder einem Balsam, dargereicht werden. Hierbei kann der Träger ein pflanzliches oder tierisches Öl, ein Mineralöl, ein synthetisches Öl oder eine Mischung solcher Öle sein.

In einer besonderen Ausführungsform der erfindungsgemäß verwendeten Mittel liegen die Mittel als Mikroemulsion vor. Unter Mikroemulsionen werden im Rahmen der Erfindung neben den thermodynamisch stabilen Mikroemulsionen auch die sogenannten "PIT"-Emulsionen verstanden. Bei diesen Emulsionen handelt es sich um Systeme mit den 3 Komponenten Wasser, Öl und Emulgator, die bei Raumtemperatur als Öl-in-Wasser-Emulsion vorliegen. Beim Erwärmen dieser Systeme bilden sich in einem bestimmten Temperaturbereich (als Phaseninversiontemperatur oder "PIT" bezeichnet) Mikroemulsionen aus, die sich bei weiterer Erwärmung in Wasser-in-Öl(W/O)-Emulsionen umwandeln. Bei anschließendem Abkühlen werden wieder O/W-Emulsionen gebildet, die aber auch bei Raumtemperatur als Mikroemulsionen oder als sehr feinteilige Emulsionen mit einem mittleren Teilchendurchmesser unter 400 nm und insbesondere von etwa 100-300 nm, vorliegen. Erfindungsgemäß können solche Mikro- oder "PIT"-Emulsionen bevorzugt sein, die einen mittleren Teilchendurchmesser von etwa 200 nm aufweisen. Einzelheiten bezüglich dieser "PIT-Emulsionen" z. B. der Druckschrift Angew. Chem. 97, 655 - 669 (1985) zu entnehmen.

Die folgenden Beispiele sollen die vorliegende Erfindung verdeutlichen, ohne sie hierauf zu beschränken.

### Ausführungsbeispiele

### Beispiel 1: Beeinflussung des Wachstums von Staphylococcus epidermidis und Staphylococcus hominis mit Pflanzenextrakten

Aus flüssigen Vorkulturen von *S. epidermidis* und *S. hominis* wurden Kulturen in LB-Medium mit einer Optischen Dichte OD (600nm) von 0,05 angeimpft. Parallel zu den Kontrollen (ohne Zusatz von Zellen bzw. Extrakt) wurden je 2 Kulturen mit 1% Pflanzenextrakt versetzt und das Wachstum über 30 h anhand der Messung der OD dokumentiert. Nach 30 h wurde die Differenz der OD der Kulturen mit Extraktzusatz zu den entsprechenden Kontrollen (ohne Extraktzusatz, bereinigt um den Wert ohne Zellen) bestimmt. Die eingesetzten Extrakte von weißem Tee (Camellia sinensis), Karkade (Hibiskus), Malve, Weintrauben, Traubenkernen, Karotte/Jojoba, Myrrhe und Calendula fördern selektiv das Wachstum von *S. epidermidis* bei gleichzeitiger Hemmung von *S. hominis.*

**Tabelle 1: Wachstumseffekte verschiedener Pflanzenextrakte auf S. epidermidis und S. hominis (Förder- bzw. Hemmfaktor nach 30 h im Vergleich zur Kontrolle)**

| | Weißer Tee | Kar-ka de | Mal-v e | Wein-tra uben | Traubenkern | Myrrhe | Karotte/ Jojoba | Calendula |
|---|---|---|---|---|---|---|---|---|
| *S. epidermidis* | 1,18 | 1,67 | 1,56 | 1,19 | 1,38 | 1,47 | 1,10 | 1,43 |
| *S. hominis* | 0,77 | 0,79 | 0,83 | 0,79 | 0,64 | 0,99 | 0,63 | 0,92 |

### Beispiel 2: Wachstumseffekte von unterschiedlich hergestellten Hibiscusextrakten (Karkade) auf S. epidermidis und S. hominis

Aus flüssigen Vorkulturen von *S. epidermidis* und *S. hominis* wurden Kulturen in LB-Medium mit einer Optischen Dichte OD (600nm) von 0,05 angeimpft. Parallel zu den Kontrollen (ohne Zusatz von Zellen bzw. Extrakt) wurden je 2 Kulturen mit 1% Pflanzenextrakt versetzt und das Wachstum Ober 30 h anhand der Messung der OD dokumentiert. Nach 30 h wurde die Differenz der OD der Kulturen mit Extraktzusatz zu den entsprechenden Kontrollen (ohne Extraktzusatz, bereinigt um den Wert ohne Zellen) bestimmt. Eingesetzt wurden unterschiedliche Hibiscus-Extrakte von Cosmetochem, nämlich "Herbasol Extrakt unkonserviert Karkade in 80 % PG", "Karkade (Hibiscus) Herbasec" sowie "Karkade (Hibiscus) Herbasol Extrakt öllöslich".

**Tabelle 2: Wachstumseffekte von unterschiedlich hergestellten Hibiscusextrakten (Karkade) auf S. epidermidis und S. hominis (Förder- bzw. Hemmfaktor nach 24 h im Vergleich zu Isopropylmyristat)**

| | Wasser/ Propylenglykol | Wasser/Ethanol (Auf Maltodextrin-Träger) | Isopropylmyristat |
|---|---|---|---|
| S. epidermidis | 1,36 | 2,59 | 1,38 |
| *S. hominis* | 1,00 | 1,00 | 1,01 |

### Beispiel 3: Wirkung von Deo-Rezepturen, die Hibiscus-Extrakt und antimikrobielle Komponenten enthalten, auf das Wachstum von Staphylococcus epidermidis und Sfaphylococcus hominis

Aus Übernachtkulturen von *S. epidermidis* und *S. hominis* wurden Ansätze mit einer OD₆₀₀=0,1 (enstpricht ca. 10⁴ Zellen/ml) in 100 ml LB-Medium hergestellt, denen zuvor Deoformulierungen ohne bzw. mit 1 Gew.-% Hibiscusextrakt in einer Endkonzentration von 10 % (v/v) zugegeben wurden. Die Kulturen wurde anschließend für insgesamt 48 h bei 37°C und 100 Upm inkubiert. Bei 0 und 48 h wurde die Keimzahl durch Ausplattieren bestimmt.

Aufgrund der antimikrobiell wirksamen Komponenten erfolgt eine unspezifische Hemmung aller Bakterienarten, jedoch wird der antibakterielle Effekt synergistisch überlagert von dem präbiotischen Effekt der präbiotisch wirksamen Substanz.

So wird aus Tabelle 5 deutlich, dass die verwendeten Deoformulierungen ohne Hibiscus-Extrakt auf *S. hominis* und *S. epidermidis* einen etwa gleich stark hemmenden Einfluss haben. Bei den Formulierungen mit Hibiscus-Extrakt zeigt sich hingegen eine deutlich schwächere Hemmung von *S. epidermidis* bei gleichbleibend starker Hemmung von *S. hominis.*

**Tabelle 5: Einfluss von Deo-Rezepturen mit und ohne Hibiscus-Extrakt im Vergleich**

| | S. epidermidis | | S. hominis | |
|---|---|---|---|---|
| Zeit (h) | ohne Hibiscus | mit Hibiscus | ohne Hibiscus | mit Hibiscus |
| 0 | 1,41E+04 | 1,41E+04 | 4,57E+04 | 4,57E+04 |
| 48 | 7,00E+02 | 3,20E+03 | 1,00E+02 | 1,00E+02 |

### Beispielrezepturen

### Wasserfreie tensidhaltige AT-Stifte (Angaben in Gewichtsteilen)

| | **1.1** | **1.2** | **1.3** | **1.4** | **1.5** | **1.9** |
|---|---|---|---|---|---|---|
| Eutanol^{®} G 16 | 10 | - | - | 15 | 10 | 10 |
| Cetiol^{®} OE | - | 10 | 15 | - | - | - |
| Ucon Fluid^{®} AP | 5 | 5 | 5 | 5 | 5 | 5 |
| Cutina^{®} HR | 6 | 6 | 6 | 6 | 6 | 6 |
| Lorol^{®} C 18 | 20 | 20 | 20 | - | 20 | 20 |
| Lanette^{®} O | - | - | - | 20 | - | - |
| Eumulgin^{®} B 3 | 3 | 3 | 3 | 3 | 3 | - |
| Cutina^{®} E 24 PF | - | - | - | - | 5 | - |
| Aluminiumchlorohydrat | 20 | 20 | 20 | 20 | 20 | - |
| Talkum | 8 | 8 | 8 | 8 | 8 | 28 |
| Präbiotisches Pflanzenextrakt | 0,4 | 0,6 | 0,8 | 1,0 | 1,2 | 0,4 |
| Sensiva^{®} SC 50 | - | - | - | - | - | - |
| Isopropylmyristat | - | - | - | - | - | 1,0 |
| Siliconöl DC^{®} 245 | ad 100 | ad 100 | ad 100 | ad 100 | ad 100 | ad 100 |

### Sprühfähige, translucente Antitranspirant-Mikroemulsionen (Angaben in Gew.-%)

| | **2.1** | **2.2** | **2.3** | **2.4** | **2.5** | **2.6** | **2.8** |
|---|---|---|---|---|---|---|---|
| Plantaren^{®} 1200 | 1,71 | 1,71 | - | 1,71 | 1,71 | - | 1.71 |
| Plantaren^{®} 2000 | 1,14 | 1,39 | 2,40 | 1,14 | 1,39 | 2,40 | 1,39 |
| Glycorinmonooleat | 0,71 | 0,71 | - | 0,71 | 0,71 | - | 0,71 |
| Dioctylether | 4,00 | 4,00 | 0,09 | 4,00 | 4,00 | 0,09 | 4,00 |
| Octyldodecanol | 1,00 | 1,00 | 0,02 | 1,00 | 1,00 | 0,02 | 1,00 |
| Parfümöl | 1,00 | 1,00 | 1,00 | 1,00 | 1,00 | 1,00 | 1,00 |
| Aluminiumchlorohydrat | 8,00 | 5,00 | 5,00 | - | - | - | 5,00 |
| 1,2-Propylenglycol | 5,00 | 5,00 | - | 5,00 | 5,00 | - | 5,00 |
| Glycerin | - | - | 5,00 - | - | - | 5,00 - | - |
| Sensiva® SC 50 | - | - | - | - | - | - | 0,5 |
| Präbiotisches Pflanzenextrakt | 0,2 | 0,4 | 0,6 | 0,8 | 1,0 | 1,2 | 0,5 |
| Wasser | ad 100 | ad 100 | ad 100 | ad 100 | ad 100 | ad 100 | ad 100 |

### Seifenhaltige Stifte (Angaben in Gew.-%)

| | **3.1** | **3.2** | **3.3** | **3.4** | **3.5** |
|---|---|---|---|---|---|
| Methanol | 22,5 | 22,5 | 22,5 | 22,5 | 22,5 |
| Cutina^{®} FS 45 | 4,4 | 4,4 | 4,4 | 4,4 | 4,4 |
| 1,3 Butandiol | 31,7 | 31,7 | 31,7 | 31,7 | 31,7 |
| 1,2 Propylenglykol | 21,0 | 21,0 | 21,0 | 21,0 | 21,0 |
| Eutanol^{®} G | 2,0 | 2,0 | 2,0 | 2,0 | 2,0 |
| Aethoxal^{®} B | 3,0 | 3,0 | 3,0 | 3,0 | 3,0 |
| Cremophor^{®} RH 455 | 0,05 | 0,05 | 0,05 | 0,05 | 0,05 |
| NaOH 45 %ig | 1,44 | 1,44 | 1,44 | 1,44 | 1,44 |
| Parfümöl | 2,0 | 2,0 | 2,0 | 2,0 | 2,0 |
| Präbiotisches Pflanzenextrakt | 0,2 | 0,4 | 0,6 | 0,8 | 1,0 |
| Sensiva® SC 50 | - | - | - | - | - |
| Isopropylmyristat | - | - | - | - | - |
| Wasser dest. | ad 100 | ad 100 | ad 100 | ad 100 | ad 100 |

### Seifenhaltige Deodorant-Stifte (Angaben in Gew.-%)

| | **3.1** | **3.2** | **3.3** | **3.4** | **3.5** | **3.6** |
|---|---|---|---|---|---|---|
| Ethanol | 22,5 | 22,5 | 22,5 | 22,5 | 22,5 | 22,5 |
| Cutina^{®} FS 45 | 4,4 | 4,4 | 4,4 | 4,4 | 4,4 | 4,4 |
| 1,3 Butandiol | 31,7 | 31,7 | 31,7 | 31,7 | 31,7 | 31,7 |
| 1,2 Propylenglycol | 21,0 | 21,0 | 21,0 | 21,0 | 21,0 | 21,0 |
| Eutanol^{®} G | 2,0 | 2,0 | 2,0 | 2,0 | 2,0 | 2,0 |
| Aethoxal^{®} B | 3,0 | 3,0 | 3,0 | 3,0 | 3,0 | 3,0 |
| Cremophor^{®} RH 455 | 0,05 | 0,05 | 0,05 | 0,05 | 0,05 | 0,05 |
| NaOH 45 %ig | 1,44 | 1,44 | 1,44 | 1,44 | 1,44 | 1,44 |
| Phenoxyethanol | 1,0 | 1,0 | 1,0 | 1,0 | 1,0 | 1,0 |
| Sensiva^{®} SC 50 | 0,3 | 0,3 | 0,3 | 0,3 | 0,3 | 0,3 |
| Parfümöl | 2,0 | 2,0 | 2,0 | 2,0 | 2,0 | 2,0 |
| Präbiotisches Pflanzenextrakt | 0,1 | 0,3 | 0,5 | 0,8 | 1,0 | 0,5 |
| Isopropylmyristat | - | - | - | - | - | 0,8 |
| Wasser dest. | ad 100 | ad 100 | ad 100 | ad 100 | ad 100 | ad 100 |

### Deodorant im Pumpzerstäuber (Angaben in Gew.-%)

| | **4.1** | **4.2** | **4.3** | **4.4** | **4.5** |
|---|---|---|---|---|---|
| Ethanol 96 %-ig, (DEP vergällt) | 55,0 | 55,0 | 55,0 | 55,0 | 55,0 |
| Triethylcitrat | 3,0 | 3,0 | 3,0 | 3,0 | 3,0 |
| Cremophor^{®} RH 455 | 0,5 | 0,5 | 0,5 | 0,5 | 0,5 |
| Präbiotisches Pflanzenextrakt | 0,2 | 0,4 | 0,6 | 0,8 | 1,0 |
| Sensiva^{®} SC 50 | - | - | - | - | - |
| Parfümöl | 1,0 | 1,0 | 1,0 | 1,0 | 1,0 |
| Wasser | ad 100 | ad 100 | ad 100 | ad 100 | ad 100 |

### Wasserfreies Deodorant-Spray (Angaben in Gew.-%)

| | **6.1** | **6.2** |
|---|---|---|
| 2-Octyldodecanol | 0,5 | 0,5 |
| Ethanol 99 %-ig, (DEP vergällt) | 39 | 39,45 |
| Präbiotisches Pflanzenextrakt | 0,5 | 1,0 |
| Sensiva® SC 50 | - | - |
| Isopropylmyristat | - | - |
| n-Butan | 60 | 60 |

### Antitranspirant Roll-on (Angaben in Gew.-%)

| | **5.1** | **5.2** | **5.3** |
|---|---|---|---|
| Ethanol 96 %-ig, (DEP vergällt) | 30,0 | 30,0 | 30,0 |
| Mergital^{®} CS 11 | 2,0 | 2,0 | 2,0 |
| Eumulgin^{®} B 3 | 2,0 | 2,0 | 2,0 |
| Aluminiumchlorohydrat | 20,0 | 20,0 | 20,0 |
| Hydroxyethylcellulose | 0,5 | 0,5 | 0,5 |
| Präbiotisches Pflanzenextrakt | 0,2 | 0,5 | 1,0 |
| Sensiva® SC 50 | - | - | - |
| Isopropylmyristat | - | - | - |
| Parfümöl | 0,8 | 0,8 | 0,8 |
| Wasser | ad 100 | ad 100 | ad 100 |

### Antitranspirant-Spray vom Suspensionstyp (Angaben in Gew.-%)

| | **6.1** | **6.2** |
|---|---|---|
| DC-245 | 10,0 | 10,0 |
| Isopropylmyristat | 5,0 | 5,0 |
| Aluminiumchlorohydrat-Pulver | 5,0 | 5,0 |
| Aerosil^{®} R 972 | 2,0 | 2,0 |
| Sensiva® SC 50 | - | - |
| Präbiotisches Pflanzenextrakt | 0,5 | 1,0 |
| n-Butan | ad 100 | ad 100 |

### Transparentes Antitranspirant-Gel (Angaben in Gew.-%)

| | | **8.1** |
|---|---|---|
| Phase 1 | DC-245 | 7,0 |
| | DC-3225 | 10,0 |
| | Präbiotisches Pflanzenextrakt | 1,0 |
| Phase 2 | Chlorhydrol^{®} | 50,0 |
| | 1,2-Propylenglycol | 16,0 |
| | Wasser | 16,9 |

Unter Rühren wird die Phase 2 mit Hilfe eines Tropfzylinders innerhalb von 25 Minuten zu Phase 1 zugegeben. Anschließend wird 30 Minuten nachgerührt. Danach wird die Masse 120 Sekunden lang durch Bewegung des Glases am Scherkopf gleichmäßig homogenisiert (Ultra Turrax T50 (Fa. IKA-Werke), Turraxstab, Stufe 8 (ca. 8500 Upm)).

### Antitranspirant- oder Deodorant-Tücher

Für die erfindungsgemäße Ausführungsform als Antitranspirant-Tuch oder Deodorant-Tuch wird ein einlagiges Substrat aus 100 % Viskose mit einem Flächengewicht von 50 g/m² mit jeweils 75 g der Beispielemulsionen 2.1 bzw. 2.2 bzw. 2.3 pro Quadratmeter oder mit jeweils 75 g der Beispiellösungen 4.1 bzw. 4.2 beaufschlagt, in Tücher geeigneter Größe geschnitten und in Sachets verpackt.

### Weitere Beispielrezepturen (Angaben in Gew.-%)

| **Deodorant Aerosol Basis 1** | |
|---|---|
| | **3** |
| Triethylcitrate | 6,00 |
| Ethylhexylglycerin | 1,50 |
| Phenoxyethanol | 0,10 |
| Parfum | 1,00 |
| Extrakt aus weißem Tee | 0,15 |
| Aroma | 0,05 |
| Isopropylmyristat | |
| Ringelblumenextrakt | |
| Karkadenextrakt | 0,30 |
| Myrrheextrakt | 0,10 |
| Mischextrakt aus Karrotte und Jojoba | |
| Malvenextrakt | |
| Hydrocarbon Propellant | 70,00 |
| Alcohol Denat. | ad. 100 |

| **Deodorant Aerosol Basis 2** | | |
|---|---|---|
| | **3** | **4** |
| Isopropylmyristate | 5,00 | 2,00 |
| Phenoxyethanol | 0,10 | 0,20 |
| Parfum | 1,00 | 0,50 |
| Extrakt aus weißem Tee | 0,05 | |
| Ethylhexylglycerin | 1,50 | 3,00 |
| Weintraubenextrakt | | |
| Ringelblumenextrakt | | |
| Karkadenextrakt | 0,15 | 0,50 |
| Hydrocarbon Propellant | 78,00 | 60,00 |
| Alcohol Denat. | ad. 100 | ad. 100 |

| **Antitranspirant Aerosol Basis 1** | |
|---|---|
| | **4** |
| Aluminum chlorohydrate | |
| Aluminum chlorohydrate activated | 10,00 |
| Disteardimonium Hectorite Propylene Carbonate | 1,20 |
| Parfum | 1,50 |
| Encapsulated Parfum / Active (Firecaps) 0,10 | |
| Ethylhexylglycerin | 0,25 |
| Isopropylmyristat | |
| Mischextrakt aus Karrotte und Jojoba | |
| Myrrheextrakt | |
| Extrakt aus weißem Tee | |
| Karkadenextrakt | 0,60 |
| Hydrocarbon Propellant | 60,00 |
| Cyclopentasiloxane/ Cylclohexasiloxane | ad. 100 |

| **Roll on Alcoholic** | | |
|---|---|---|
| | **3** | **4** |
| Alcohol Denat. | 28,00 | 30,00 |
| Aluminum Chlorhydrate 50% solution | 16,00 | 16,00 |
| Aluminum Zirconium Pentachlorohydrate 40% solution | | |
| Ceteareth-12 | 2,00 | 2,00 |
| Ceteareth-30 | 1,50 | 2,00 |
| PEG-40 Hydrogenated Cator Oil | | |
| Parfum | 1,50 | 1,20 |
| Tocopheryl Acetate | | 0,25 |
| Hydroxyethylcellulose | 0,40 | 0,60 |
| Zinc Gluconate | | |
| Colours approved for Cosmetics | 0,0005 | 0,0100 |
| Weintraubenkernextrakt | | |
| Karkadenextrakt | 0,30 | 0,60 |
| Ethylhexylglycerin | | |
| Aqua | ad 100 | ad 100 |

| **Deo Stick / Seifenbasis - alcoholic** | | |
|---|---|---|
| | **1** | **3** |
| Alcohol Denat. | 40,00 | 35,00 |
| Propylene Glycol 1,2 | 30,32 | 32,32 |
| Butylene Glycol 1,3 | 12,00 | 15,00 |
| Sodium Palmitate | 3,10 | 2,80 |
| Sodium Stearate | 3,10 | 2,80 |
| Glycerin 86% | 2,00 | 1,70 |
| PPG-5-Laureth-5 | 0,50 | 1,00 |
| Parfum | 1,00 | 1,30 |
| Octyldodecanol | 1,00 | 1,00 |
| Phenoxyethanol | 1,00 | 1,00 |
| Ethylhexylglycerin | 0,50 | 0,30 |
| Tocopheryl acetate | 0,05 | 0,10 |
| PEG-40 Hydrogenated Castor Oil Isopropylmyristat | 0,02 | |
| Karkadenextrakt | 0,20 | 0,30 |
| Malvenextrakt | | 0,20 |
| Mischextrakt aus Karotte und Jojoba | | |
| Aqua | ad. 100 | ad. 100 |

| **Deo Stick / Seifenbasis - nonalcoholic** | |
|---|---|
| | **3** |
| PEG-8 | 50,00 |
| Sodium Palmitate | 2,50 |
| Sodium Stearate | 2,50 |
| Butylene Glycol 1,3 | 3,00 |
| PEG-14 Dimethicone | 1,50 |
| Phenoxyethanol | 0,50 |
| Parfum | 0,80 |
| Ehtylhexylglycerin | 0,30 |
| Steareth-10 | 0,20 |
| Mischextrakt aus Karotte und Jojoba | |
| Malvenextrakt | |
| Karkadenextrakt | 0,30 |
| Isopropylmyristat | |
| Aqua | ad. 100 |

| **Antiperspirant Stick Base 1** | |
|---|---|
| | **3** |
| PPG-14 Butylether | 12,00 |
| Hydrogenated Castor Oil | 2,00 |
| Stearyl alcohol | 15,00 |
| Ceteareth-30 | 4,00 |
| Isoceteth-20 | |
| Parfum | 0,80 |
| Aluminumchlorohydrate | 18,00 |
| Aluminium Zirconium tetrachlorohydrate Glyc | |
| Allantoin | |
| Cocoglycerides | 3,00 |
| Talc | 5,00 |
| Tocopherol acetate | 0,50 |
| Ethylhexylglycerin | |
| Isopropylmyristat | |
| Karkadenextrakt | 0,35 |
| Malvenextrakt | |
| Cyclopentasiloxane | ad 100 |

| **Antiperspirant Stick Base 2** | |
|---|---|
| | **3** |
| Hexyldecanol | 10,00 |
| PPG-14 Butylether | 6,00 |
| Hydrogenated Castor Oil | 6,00 |
| Stearyl alcohol | 11,00 |
| Cetyl alcohol | 6,00 |
| PEG-20 Glyceryl stearate | 6,00 |
| Ceteareth-30 | 3,00 |
| Parfum | 0,80 |
| Aluminumchlorohydrate | 18,00 |
| Aluminium Zirconium tetrachlorohydrate Glyc | |
| Talc | 8,00 |
| Ethylhexylglycerin | |
| Tocopherol acetate | 0,50 |
| Extrakt aus weißem Tee | |
| Isopropylmyristat | 1,00 |
| Karkadenextrakt | 0,10 |
| Cyclopentasiloxane | ad 100 |

| **Deodorant Pumpdispenser** | |
|---|---|
| | **2** |
| Alcohol Denat. | 55,00 |
| Triethylcitrate | 3,50 |
| PEG-40 Hydrogenated Castor Oil | 0,50 |
| Ethylhexylglycerin | 0,30 |
| Tocopheryl Acetate | 0,20 |
| Benzophenone-2 | 0,01 |
| Colours approved for Cosmetics | 0,0005 |
| Parfum | 1,00 |
| Weintraubenkernextrakt | |
| Myrrheextrakt | 0,15 |
| Karkadenextrakt | 0,15 |
| Ringelblumenextrakt | |
| Extrakt aus weißemTee | |
| Aqua | ad. 100 |

| **Deodorant Tuch** | |
|---|---|
| | **1** |
| Alcohol Denat. | 50,00 |
| Triethylcitrate | 2,50 |
| PEG-40 Hydrogenated Castor Oil | 1,00 |
| Ethylhexylglycerin | 0,10 |
| Tocopheryl Acetate | 0,05 |
| Benzophenone-2 | 0,01 |
| Colours approved for Cosmetics | 0,0001 |
| Parfum | 0,80 |
| Weintraubenkernextrakt | 0,25 |
| Malvenextrakt | |
| Karkadenextrakt | 0,15 |
| Weintraubenextrakt | |
| Ringelblumenextrakt | |
| Aqua | ad. 100 |

| **Antiperspirant Tuch (PIT Technology)** | | |
|---|---|---|
| | **1** | **3** |
| Aluminum chlorohydrate 50% solution | 30,00 | 35,00 |
| Dicaprylyl Ether | 10,00 | 8,00 |
| Glycerin 86% | 5,00 | 5,00 |
| Beheneth-10 | 3,30 | 3,50 |
| Cetearyl lsononanoate | | 4,00 |
| Hexyldecanol / Hexyldecyl Laurate | 3,00 | |
| parfum | 1,00 | 1,20 |
| Polysorbate 20 / Linoleic Acid | 0,20 | 0,50 |
| Allantoin | 0,10 | |
| Weintraubenextrakt | 0,25 | |
| Karkadenextrakt | 0,25 | 0,50 |
| Malvenextrakt | | |
| Preservative System | 0,50 | 1,00 |
| Aqua | ad.100 | ad.100 |

| **Klares Antitranspirant Gel** | |
|---|---|
| | **3** |
| Propylene Glycol 1,2 Aluminum Chlorohydrate | **18,00** |
| 50% solution | 40,00 |
| Cyclopentasiloxane | 14,20 |
| Alcohol Denat. | 8,00 |
| BIS-PEG/PPG-14/14 Dimethicone | 3,20 |
| Parfum | 1,00 |
| Allantoin | |
| Ethylhexylglycerin | |
| Ringeiblumenextrakt | |
| Weintraubenkernextrakt | |
| Karkadenextrakt | 0,50 |
| Myrrheextrakt | |
| Aqua | ad.100 |

| **Klares Deodorant Gel** | |
|---|---|
| | **3** |
| Alcohol Denat. | **50,00** |
| Ceteareth-12 | 2,00 |
| Ceteareth-30 | 2,00 |
| PEG-40 Hydrogenated Castor Oil Carbomer | 0,80 |
| Parfum | 1,00 |
| Malvenextrakt | |
| Myrrhenextrakt | |
| Weintraubenkernextrakt | |
| Mischextrakt aus Karotte und Jojoba | |
| Karkadenextrakt | 0,35 |
| Aqua | ad. 100 |

Um klare Gele zu erzielen, ist der Brechungsindex der Wasserphase dem Brechungsindex der Ölphase anzupassen, Wasser bzw. Propylenglycol dienen als Variable.

Der Verdicker (Carbomer) ist mit einem geeigneten Neutralisierungmittel (TEA, AMP, NaOH, LiOH) auf den gewünschten pH Wert einzustellen.

| **Antiperspirant Cream** | | | |
|---|---|---|---|
| | **2** | **3** | **4** |
| Aluminum Chlorohydrate 50% solution 40,00 | | 35,00 | 45,00 |
| Glyceryl Stearate | 4,50 | 5,50 | 6,00 |
| Cetyl Alcohol | 1,50 | 3,00 | 1,50 |
| Behenyl Alcohol | 4,00 3,50 | | 5,00 |
| Dimethicone | 1,50 | 2,50 | 3,00 |
| Ceteareth-12 | 2,00 | 2,50 | 1,30 |
| Ceteareth-20 | 2,00 | 2,50 | 1,30 |
| Hexyldecanol / Hexyldecyl Laurate | 4,00 | 2,50 | 2,40 |
| Cyclopentasiloxane | 3,00 | 2,00 | 1,00 |
| Tocopheryl Acetate | 0,25 | | |
| Parfum | 1,00 | 1,50 | 2,00 |
| Allantoin | 0,10 | | |
| Perservative System | 0,50 | 0,50 | 0,50 |
| Ethylhexylglycerin | | | |
| Isopropylmyristat | 1,0 | | |
| Karkadenextrakt | 0,2 | 0,5 | 0,8 |
| Aqua | ad. 100 | ad.100 | ad.100 |

| **Deodorant / Antitranspirant Puder** | | |
|---|---|---|
| | **3** | **4** |
| | | |
| Aluminum Chlorohydrate | | |
| Aluminium Zirconium tetrachlorohydrate Glyc | | 24,00 |
| Silica | 1,00 | 1,00 |
| Triclosan | 0,10 | |
| Sensiva SC 50 | 1,00 | |
| Parfum | 2,00 | 1,00 |
| Extrakt aus grünem Tee | 0,50 | |
| Extrakt aus weißem Tee | | |
| Weintraubenextrakt | 0,5 | |
| Karkadenextrakt | 0,1 | 0,6 |
| Talc | ad. 100 | ad. 100 |

| **Deodorant Seife** | |
|---|---|
| | **4** |
| Sodium Tallowate | |
| Sodium Palmitate | 60,00 |
| Sodium Cocoate | |
| Sodium Palm Oleate | 27,00 |
| Talc | |
| Lauryl Glucoside | 2,00 |
| Parfum | 0,50 |
| Sodium Chloride | 0,50 |
| Tetrasodium EDTA | 0,10 |
| Tocopherol | |
| Antibacterial Active | |
| Colours approved for Cosmetics | |
| Ethylhexylglycerin | |
| Isopropylmyristat | |
| Karkadenextrakt | 0,6 |
| Aqua | ad. 100 |

| **Deodorant Wasch Lotion** | |
|---|---|
| | **4** |
| Sodium Laureth Sulfate | |
| Disodium Laureth Sulfosuccinate | 8,00 |
| Lauryl Glucoside | 4,00 |
| Potassium cocoyl Hydrolyzed Collagen 2,00 | |
| PEG-7 Glyceryl Cocoate | 3,00 |
| PEG-120 Methyl Glucose Dioleate | 1,00 |
| Parfum | 0,50 |
| Tetrasodium EDTA | 0,10 |
| Tocopherol Acetate | |
| Antibacterial | |
| Citric Acid | 0,30 |
| Colours approved for Cosmetics | |
| Ethylhexylglycerin | |
| Traubenkernextrakt | |
| Karkadenextrakt | 0,8 |
| Aqua | ad. 100 |

### Liste der eingesetzten Rohstoffe

| **Rohmaterial** | **Lieferant** | **INIC-Nomenklatur** |
|---|---|---|
| Allantoin | Merck | Allantoin |
| Rezal G Solution | Reheis | Aluminium Tetrachlorohydrate Glyc 35% solution |
| Rezal 36 GP SUF | Reheis | Aluminium Zirconium tetrachlorohydrate Glyc |
| Locron L | Clariant | Aluminum Chlorhydrate 50% solution |
| Microdry | Reheis | Aluminum chlorohydrate |
| Microdry UF | Reheis | Aluminum Chlorohydrate |
| Chlorhydrol Solution | Reheis | Aluminum chlorohydrate 50% solution |
| Reach 103 | Reheis | Aluminum chlorohydrate activated |
| Rezal 67 | Reheis | Aluminum Zirconium Pentachlorohydrate 40% solution |
| Dry Flo PC | National Starch | Aluminum-Starch-Octenylsuccinate |
| Cooling Agent | Different | Aroma |
| Mergital B10 | Cognis | Beheneth-10 |
| Stenol 1822 A | Cognis | Behenyl Alcohol |
| Uvinul D 50 | BASF | Benzophenone-2 |
| Abil EM 97 | Degussa | BIS-PEG/PPG-14/14 Dimethicone |
| Syncrowax HGLC | Croda | C18-C36 Triglyceride |
| Carbopol ETD 2001 | Noveon | Carbomer |
| Vitacel L-600-20 FCC | Rettenmaier | Cellulose |
| Mergital CS 11 | Cognis | Ceteareth-11 |
| Eumulgin B1 | Cognis | Ceteareth-12 |
| Eumulgin B2 | Cognis | Ceteareth-20 |
| Eumulgin B3 | Cognis | Ceteareth-30 |
| Lanette O | Cognis | Cetearyl Alcohol |
| Cetiol SN | Cognis | Cetearyl Isononanoate |
| Lorol C16 | Cognis | Cetyl Alcohol |
| Novata AB | Cognis | Cocoglycerides |
| dc 245 | Dow Corning | Cyclopentasiloxane |
| Dc 3225 | Dow Corning | Cyclomethicone / Dimethicone Copolyol |
| dc 345 | Dow Corning | Cyclopentasiloxane/ Cylclohexasiloxane |
| Cosmacol EMI | Condea | Di-C12-13 Alkyl Malate |
| Cetiol OE | Cognis | Dicaprylether |
| Baysilone M350 | Bayer | Dimethicone |
| Powder Quality | Different | Disodium Laureth Sulfosuccinate |
| Texapon SB 3 UP | Cognis | Disodium Laureth Sulfosuccinate |
| Bentone Gel VS 5 PCV | Rheox | Dlsteardimonium Hectorite Propylene Carbonate |
| Fircaps | Firmenich | Encapsulated Parfum / Active (Fircaps) |
| Sensiva SC 50 | Schülke & Mayr | Ethylhexylglycerin |
| Cegesoft C24 | Cognis | Ethylhexylpalmitat |
| Cutina MD-V | Cognis | Glyceryl Stearate |
| Eutanol G 16 | Cognis | Hexyldecanol |
| Cetiol PGL | Cognis | Hexyldecanol / Hexyldecyl Laurate |
| Drivosol | Hüls | Hydrocarbon Propellant |
| Cutina HR | Cognis | Hydrogenated Castor Oil |
| Natrosol 250 HR | Hercules Aqualon | Hydroxyethylcellulose |
| Arlasolv 200 | Uniqema | Isoceteth-20 |
| Isopropylmyristate | Cognis | Isopropylmyristate |
| Plantacare 1200 UP | Cognis | Lauryl Glucoside |
| Eutanol G | Cognis | Octyldodecanol |
| Parfum | Different | Parfum |
| Glucamte DOE 120 | Amerchol | PEG-120 Methyl Glucose Dioleate |
| Abil B 8843 | Degussa | PEG-14 Dimethicone |
| Cutina E 24 | Cognis | PEG-20 Glycerylstearate |
| Eumulgin HRE 40 | Cognis | PEG-40 Hydrogenated Castor Oil |
| Cetiol HE | Cognis | PEG-7 Glyceryl Cocoate |
| Phenoxyethanol | Bayer | Phenoxyethanol |
| Plant Extract | Different | Plant Extract |
| Vitamin F watersoluble | Crodarom | Polysorbate 20 / Linoleic Acid |
| Lamepon S | Cognis | Potassium Cocoyl Hydrolyzed Collagen |
| Ucon Fluid AP | Ucon | PPG-14 Butylether |
| Arlamol E | Uniqema | PPG-15 Stearyl Ether |
| Aethoxal B | Cognis | PPG-5-Laureth-5 |
| Aerosil 200 | Degussa | Silica |
| Aerosil 200 | Degussa | Silica |
| Aerosol R 972 | Degussa | Silica Dimethyl Silylate |
| Soap based on Edenor K12-18 | Cognis | Sodium Cocoate |
| Powder Quality | Different | Sodium Laureth Sulfate |
| Texapon NSO UP | Cognis | Sodium Laureth Sulfate |
| Soap based on Edenor | Cognis | Sodium Palm Oleate |
| Soap based on Cutina FS 45 | Cognis | Sodium Palmitate |
| Soap based on Edenor C16-96/100 | Cognis | Sodium Palmitate |
| Soap based on Cutina FS 45 | Cognis | Sodium Stearate |
| Soap based on Edenor TIS GAH | Cognis | Sodium Tallowate |
| Powder Quality | Different | Sodiumcocoylisethionate |
| Brij 76 | Uniqema | Steareth-10 |
| Brij 72 | Uniqema | Steareth-2 |
| Brij 721 | Uniqema | Steareth-21 |
| Lorol C18 | Cognis | Stearyl alcohol |
| Lorol C18 | Cognis | Stearyl alcohol |
| Steasilk 5 GGHT | Luzenac | Talc |
| Talkum Pharma G | Grolmann | Talc |
| Trilon B fl. | BASF | Tetrasodium EDTA |
| Vitamin E | Roche | Tocopherol |
| Vitamin E Acetate | BASF | Tocopheryl Acetate |
| Irgasan DP 300 | Ciba Spec. | Triclosan |
| Citrofol AL | Jungbunzlauer | Triethylcitrate |
| Zinkgluconat | Interorgana | Zinc Gluconate |

| | | |
|---|---|---|
| Blätterextrakt aus Weißem Tee | | Cosmetochem |
| Karkaden-Extrakt | | Cosmetochem |
| Blütenextrakt aus Malve | | Cosmetochem |
| Weintrauben-Extrakt | | Cosmetochem |
| Mischextrakt aus Karotte und Jojoba | | Cosmetochem |
| Myrrhe-Extrakt | | Cosmetochem |
| Ringelblumen-Extrakt | | Cosmetochem |
| Traubenkernextrakt | | Cosmotechem |
| Güner Tee-Extrakt | | Dragoco |

## Patentansprüche

1. Nicht-therapeutische Verwendung von auf der Haut präbiotisch wirksamen Substanzen, in Form von kosmetischen topischen Hautbehandlungsmitteln, zur Förderung des Wachstums erwünschter Hautkeime und zur Hemmung des Wachstums unerwünschter Hautkeime zur Behandlung von Körpergeruch, **dadurch gekennzeichnet, dass** die präbiotisch wirksame. Substanz ein Wasser/Ethanol-Extrakt aus Hibiscus sabdariffa ist.

2. Verwendung nach Anspruch 1, **dadurch gekennzeichnet, dass** das Hautbehandlungsmittel mindestens eine weitere Substanz enthält, die ausgewählt ist aus mindestens einer der folgenden Gruppen:
- oberflächenaktive Substanzen als Emulgator oder Dispergiermittel,
- Aminosäuren und deren Zinksalze und deren Säureadditionssalze,
- filmbildende und/oder emulsionsstabilisierende und/oder verdickende und/oder adhäsive Polymere,
- Fettstoffe, Tenside, Anti-Transpirantien und Polyole,
- organische, mineralische und modifizierte mineralische Lichtschutzfilter,
- Proteinhydrolysate und deren Derivaten,
- Mono-, Oligo- und Polysaccharide sowie deren Derivate,
- α-Hydroxycarbonsäuren und α-Ketocarbonsäuren sowie deren Ester-, Lacton- oder Salzformen.

3. Verwendung nach Anspruch 1 oder 2, **dadurch gekennzeichnet, dass** das Hautbehandlungsmittel in einer Seife, einer Lotion, einem Puder, einem Syndet, einem Schaum, einem Stift, einer Emulsion, einem Spray, einer Creme, einem Gel, einem Shampoo oder einem Pflaster enthalten ist.

4. Verwendung nach Anspruch 1, **dadurch gekennzeichnet, dass** der Extrakt auf einen Talk-Träger oder einen Maltodextrin-Träger aufgebracht ist.

## Claims

1. Non-therapeutic use of substances with a prebiotic effect on the skin, in the form of cosmetic topic skin treatment agents, for promoting growth of desirable skin germs and for inhibiting growth of undesirable skin germs, for treating body odour, **characterized in that** the substance with a prebiotic effect is a water/ethanol extract of Hibiscus sabdariffa.

2. The use according to claim 1, **characterized in that** the skin treatment agent contains at least one further substance which is selected from at least one of the following groups:
- surfactant substances as an emulsifier or dispersant,
- aminoacids and their zinc salts and their acid addition salts,
- film-forming and/or emulsion-stabilizing and/or thickening and/or adhesive polymers,
- fats, surfactants, antiperspirants and polyols,
- organic, mineral and modified mineral light protection filters,
- protein hydrolyzates and their derivatives,
- mono-, oligo- and poly-saccharides as well as their derivatives,
- α-hydroxycarboxylic acids and α-ketocarboxylic acids as well as their ester, lactone or salt forms.

3. The use according to claim 1 or 2, **characterized in that** the skin treatment agent is contained in a soap, a lotion, a powder, a syndet, a foam, a stick, an emulsion, a spray, a cream, a gel, a shampoo or a patch.

4. The use according to claim 1, **characterized in that** the extract is applied on talc carrier or a maltodextrin carrier.

## Revendications

1. Utilisation non thérapeutique de substances à activité prébiotique sur la peau, sous la forme d'agents de traitement cutané cosmétiques par voie locale, pour favoriser la croissance de germes cutanés désirés et pour inhiber la croissance de germes cutanés non désirés pour le traitement d'odeurs corporelles, **caractérisée en ce que** la substance à activité prébiotique est un extrait aqueux/éthanolique d'Hibiscus sabdariffa.

2. Utilisation selon la revendication 1, **caractérisée en ce que** l'agent de traitement cutané contient au moins une substance supplémentaire qui est choisie parmi au moins un des groupes suivants :
- des substances tensioactives à titre d'émulsifiants ou d'agents de mise en dispersion ;
- des acides aminés et leurs sels de zinc et leurs sels d'addition d'acides ;
- des polymères filmogènes et/ou stabilisateurs de l'émulsion et/ou épaississants et/ou adhésifs ;
- des matières grasses, des agents tensioactifs, des agents antitranspirants et des polyols ;
- des filtres organiques, minéraux et minéraux modifiés de protection contre l'effet de la lumière ;
- des hydrolysats protéiniques et leurs dérivés ;
- des monosaccharides, des oligosaccharides et des polysaccharides ainsi que leurs dérivés ;
- des acides α-hydroxycarboxyliques et des acides α-cétocarboxyliques ainsi que leurs formes d'esters, de lactones ou de sels.

3. Utilisation selon la revendication 1 ou 2, **caractérisée en ce que** l'agent de traitement cutané est contenu dans un savon, dans une lotion, dans une poudre, dans un syndet, dans une mousse, dans un bâton, dans une émulsion, dans un spray, dans une crème, dans un gel, dans un shampooing ou dans un emplâtre.

4. Utilisation selon la revendication 1 ou 2, **caractérisée en ce que** l'extrait est appliqué sur un support à base de talc ou sur un support à base de maltodextrine.
